# EUROPEAN PATENT SPECIFICATION

(11) **EP 4 162 046 B1**
(45) Date of publication and mention of the grant of the patent: **16.10.2024**
(21) Application number: 21730620.8
(22) Date of filing: 09.06.2021
(51) Int. Cl.: C12N 15/113, C07K 14/47, A61P 9/00

(54) **TREATMENT METHOD OF LEFT VENTRICULAR DYSFUNCTION FOLLOWING AN ACUTE MYOCARDIAL INFARCTION**
THERAPIE ZUR VERHINDERUNG UNERWÜNSCHTER KARDIALER REMODELLIERUNG NACH EINEM AKUTEN MYOKARDINFARKT
THÉRAPIE POUR EMPÊCHER UN REMODELAGE CARDIAQUE INDÉSIRABLE APRÈS UN INFARCTUS AIGU DU MYOCARDE

(30) Priority: 09.06.2020 EP 20382498
(43) Date of publication of application: 12.04.2023
(73) Proprietor: Universidad de Murcia, 30072 Murcia (ES)
(72) Inventor: PASCUAL FIGAL, Domingo Andrés, 30072 Murcia (ES); LAX PÉREZ, Antonio Manuel, 30072 Murcia (ES); ASENSIO LÓPEZ, María Carmen, 30072 Murcia (ES)
(74) Representative: Hoffmann Eitle
(86) International application number: PCT/EP2021/065521
(87) International publication number: WO 2021/250124

(56) References cited:
- ES-A1- 2 637 032
- ASENSIO-LOPEZ M C ET AL: "Yin-Yang 1 transcription factor modulates ST2 expression during adverse cardiac remodeling post-myocardial infarction", JOURNAL OF MOLECULAR AND CELLULAR CARDIOLOGY, vol. 130, 15 April 2019 (2019-04-15), pages 216 - 233, XP085676065, ISSN: 0022-2828, DOI: 10.1016/J.YJMCC.2019.04.009
- SUCHAROV CARMEN C ET AL: "Yin Yang 1 is increased in human heart failure and represses the activity of the human alpha-myosin heavy chain promoter", JOURNAL OF BIOLOGICAL CHEMISTRY, AMERICAN SOCIETY FOR BIOCHEMISTRY AND MOLECULAR BIOLOGY, US, vol. 278, no. 33, 15 August 2003 (2003-08-15), pages 31233 - 31239, XP002426318, ISSN: 0021-9258, DOI: 10.1074/JBC.M301917200
- ST JOHN SUTTON M G ET AL: "Left ventricular remodeling after myocardial infarction: pathophysiology and therapy", CIRCULATION, AMERICAN HEART ASSOCIATION, US, vol. 101, 27 June 2000 (2000-06-27), pages 2981 - 2988, XP002714260, ISSN: 0009-7322
- PASCUAL-FIGAL DOMINGO A ET AL: "The Biology of ST2: The International ST2 Consensus Panel", AMERICAN JOURNAL OF CARDIOLOGY, CAHNERS PUBLISHING CO., NEWTON, MA, US, vol. 115, no. 7, 23 January 2015 (2015-01-23), XP029203436, ISSN: 0002-9149, DOI: 10.1016/J.AMJCARD.2015.01.034
- TAN CHIA YEE ET AL: "Yin Yang 1 Suppresses Dilated Cardiomyopathy and Cardiac Fibrosis Through Regulation of Bmp7 and Ctgf", CIRCULATION RESEARCH, vol. 125, no. 9, 11 October 2019 (2019-10-11), US, pages 834 - 846, XP055793057, ISSN: 0009-7330, DOI: 10.1161/CIRCRESAHA.119.314794

## Description

### Technical field of the invention

The present invention refers to the biomedical field, in particular to a treatment method of left ventricular dysfunction following an acute myocardial infarction.

### Background of the invention

In patients suffering an acute myocardial infarction (AMI), opening the occluded coronary artery is the best strategy to recover oxygen supply and to limit ischemic myocardial damage. However, the reperfusion therapy based on primary angioplasty and/or fibrinolytics, is effective only when performed within the first 12 hours after symptoms onset [1]. Unfortunately, a significant portion of patients does not access to the reperfusion therapy in a timely manner, and every 30 min of delay is associated with a relative increase of 7.5% in death risk at one year [2]. This excess of mortality is explained because ischemic time duration is a major determinant of infarct size, which leads to pathological changes in cardiac function and structure. These pathological processes are known as adverse cardiac remodeling and include contractile dysfunction, chamber dilatation and myocardial abnormalities [3, 4]. The extension of these processes determines the progression to heart failure (HF), ventricular arrhythmias and death in the short- and long-term follow-up. Therefore, the search for new therapies in order to minimize adverse myocardial remodeling and cardiac complications following AMI are a priority in cardiovascular medicine.

Soluble suppression of tumorigenesis 2 (sST2) is a member of the interleukin 1 receptor family, also known as interleukin 1 receptor-like 1 (IL1RL1) [5] with two main isoforms: a membrane-bound receptor (ST2 ligand [ST2L]) and a soluble ST2 isoform (sST2) [4]. sST2 is a unique biomarker associated with pathological cardiac processes [6,7]. In particular, in patients suffering an AMI, circulating concentrations of sST2 have repeatedly identify a higher risk of death and the progression of adverse myocardial remodeling to HF in the short-term (30 days) and also in the long-term follow-up [8-13].

Interleukin-33 (IL-33), by interacting with ST2L [14] triggers a cardioprotective, anti-remodeling response [7, 15] that is associated with the blocking of both IκBα phosphorylation as well as the activation of NF-κB promoter activity [16]. Increased concentrations of sST2 in the circulation can bind to IL-33 directly and act as a decoy receptor, by inhibiting its binding to membrane-bound ST2L, thus blocking the cardioprotective effects of IL-33; such inhibition results in cardiac hypertrophy, myocardial fibrosis, and ventricular dysfunction [7, 15]. Several experimental studies, have shown that the expression of IL-33 and sST2 in both cardiac fibroblasts and cardiomyocytes increased in response to cardiac stress. Weinberg et al., in 2002, determined that ST2 was the most highly induced transcript in response to biomechanical stress, and the sST2 and ST2L forms were induced in neonatal cardiomyocytes subjected to cyclic strain [10]. Accordingly, using a myocardial infarct model we have shown that an increase in myocardium sST2 levels after 4 weeks positively correlates with cardiac remodeling markers, such as inflammatory and fibrosis markers [17]. Therefore, to favorably regulate the IL-33/ST2L pathway has been suggested as a therapeutic option [18].

Although we know that soluble and membrane forms of ST2 are expressed through cellular-specific modulation of a dual promoter [10], the molecular elements related to the specific sST2 expression following AMI are unknown. Using computational genomics and neonatal cardiomyocytes under biomechanical strain, we identified Yin yang-1 (Yy1) as a transcription factor related to cardiac expression of sST2 [19]. In this *in vitro* study, the silencing of the endogenous Yy1 expression resulted in a decrease in the expression and release of sST2 [19]. Altogether, these findings support the idea that reducing Yy1 expression levels would be a promising anti-adverse cardiac remodeling therapy following AMI.

The present invention provides a new therapy to improve cardiac dysfunction and adverse myocardial remodeling following AMI.

The references to methods of treatment in this description are to be interpreted as references to the compounds, pharmaceutical compositions and medicaments of the present invention for use in a method for treatment of the human (or animal) body by therapy (or for in vivo diagnosis).

### References

1. Ibanez B, James S, Agewall S, Antunes MJ, Bucciarelli-Ducci C, Bueno H, Caforio ALP, Crea F, Goudevenos JA, Halvorsen S, Hindricks G, Kastrati A, Lenzen MJ, Prescott E, Roffi M, Valgimigli M, Varenhorst C, Vranckx P, Widimský P, Baumbach A, Bugiardini R, Coman IM, Delgado V, Fitzsimons D, Gaemperli O, Gershlick AH, Gielen S, Harjola VP, Katus HA, Knuuti J, et al. 2017 ESC Guidelines for the management of acute myocardial infarction in patients presenting with ST-segment elevation. Eur Heart J 2018;39:119-177.
2. De Luca G, Suryapranata H, Ottervanger J P.et al Time delay to treatment and mortality in primary angioplasty for acute myocardial infarction: every minute of delay counts. Circulation 2004;109:1223-1225.
3. Sutton MG, Sharpe N. Left ventricular remodeling after myocardial infarction: Pathophysiology and therapy. Circulation 2000;101:2981-2988.
4. Konstam MA, Kramer DG, Patel AR, Maron MS, Udelson JE. Left ventricular remodeling in heart failure: Current concepts in clinical significance and assessment. JACC Cardiovasc Imaging 2011;4:98-108.
5. Garlanda C, Dinarello CA, Mantovani A. The interleukin-1 family: back to the future. Immunity 2013;39:1003-18.
6. Pascual-Figal DA, Januzzi JL. The Biology of ST2: The International ST2 Consensus Panel. Am. J. Cardiol. 2015;115:3B-7B.
7. Pascual-Figal DA, Lax A, Perez-Martinez MT, et al. Clinical relevance of sST2 in cardiac diseases. Clin Chem Lab Med 2016;54:29-35.
8. Weir RAP, Miller AM, Murphy GEJ, et al. Serum soluble ST2: a potential novel mediator in left ventricular and infarct remodeling after acute myocardial infarction. J Am Coll Cardiol 2010;55:243-50.
9. Bière L, Garcia G, Guillou S, et al. ST2 as a predictor of late ventricular remodeling after myocardial infarction. Int J Cardiol 2018;259:40-42.
10. Weinberg EO, Shimpo M, De Keulenaer GW, et al. Expression and regulation of ST2, an interleukin-1 receptor family member, in cardiomyocytes and myocardial infarction. Circulation 2002;106:2961-6.
11. Sabatine MS, Morrow DA, Higgins LJ, et al. Complementary roles for biomarkers of biomechanical strain ST2 and N-terminal prohormone B-type natriuretic peptide in patients with ST-elevation myocardial infarction. Circulation 2008;117:1936-44.
12. Shimpo M, Morrow DA, Weinberg EO, et al. Serum levels of the interleukin-1 receptor family member ST2 predict mortality and clinical outcome in acute myocardial infarction. Circulation 2004;109:2186-90.
13. Jenkins WS, Roger VL, Jaffe AS, et al. Prognostic Value of Soluble ST2 After Myocardial Infarction: A Community Perspective. Am J Med 2017;130:1112.e9-1112.e15.
14. Schmitz J, Owyang A, Oldham E, et al. IL-33, an interleukin-1-like cytokine that signals via the IL-1 receptor-related protein ST2 and induces T helper type 2-associated cytokines. Immunity 2005;23:479-90.
15. Seki K, Sanada S, Kudinova AY, et al. Interleukin-33 Prevents Apoptosis and Improves Survival After Experimental Myocardial Infarction Through ST2 Signaling. Circ. Hear. Fail. 2009;2:684-691.
16. Sanada S, Hakuno D, Higgins LJ, Schreiter ER, McKenzie ANJ, Lee RT. IL-33 and ST2 comprise a critical biomechanically induced and cardioprotective signaling system. J. Clin. Invest. 2007;117:1538-49.
17. Sanchez-Mas J, Lax A, Asensio-Lopez M, et al. Modulation of IL-33/ST2 system in post-infarction heart failure: correlation with cardiac remodeling markers. Eur. J. Clin. Invest. 2014;44:643-51.
18. Kakkar R, Lee RT. The IL-33/ST2 pathway: therapeutic target and novel biomarker. Nat Rev Drug Discov 2008;7:827-40.
19. Asensio-Lopez MC, Lax A, Fernandez del Palacio MJ, et al. Yin-Yang 1 transcription factor modulates ST2 expression during adverse cardiac remodeling post-myocardial infarction. J. Mol. Cell. Cardiol. 2019;130:216-233.

ES2637032-A1 discloses the use of antisense RNA compositions to down-regulate the expression of the Yin Yang gene 1, which results in the downregulation of ST2. This allows the activation of the IL33/ST2L cardioprotective signalling chain. Experimental models use metformin to test the effects in preventing cardiac remodeling after a myocardial infarction.

SUCHAROV CC et al., (2003), JOURNAL OF BIOLOGICAL CHEMISTRY, vol. 278 (33), pages 31233-31239, discloses that Yin Yang 1 expression is increased in human heart failure and represses the activity of the human alpha-myosin heavy chain promoter.

TAN CHIA YEE et al., (2019) CIRCULATION RESEARCH, vol. 125 (9), pages 834-846, discloses that Yin Yang 1 suppresses dilated cardiomyopathy and cardiac fibrosis through regulation of Bmp7 and Ctgf.

### Brief description of the figures

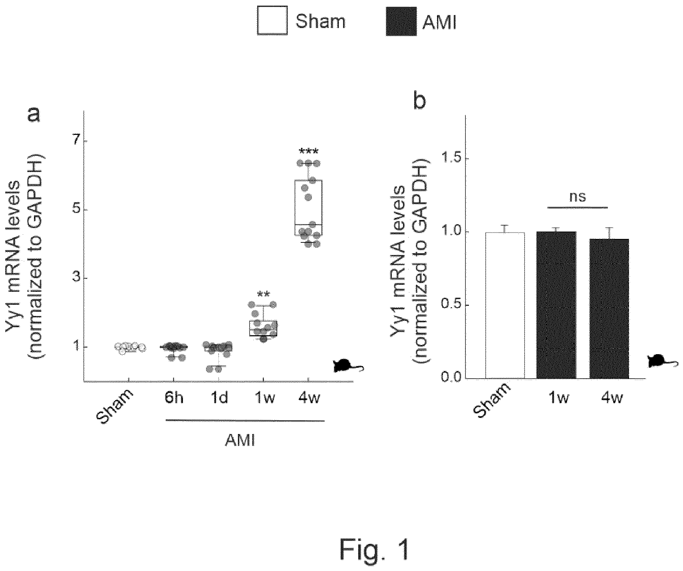

**Figure 1****.-** Yy1 expression levels increases in the infarcted LV area. (a) Yy1 mRNA levels; data were normalized to GAPDH mRNA levels. (b) Yy1 mRNA levels in the remote LV area; data were normalized to GAPDH mRNA levels. ** p<0.01, *** p<0.001 in compared to sham group. ns: non-significant.

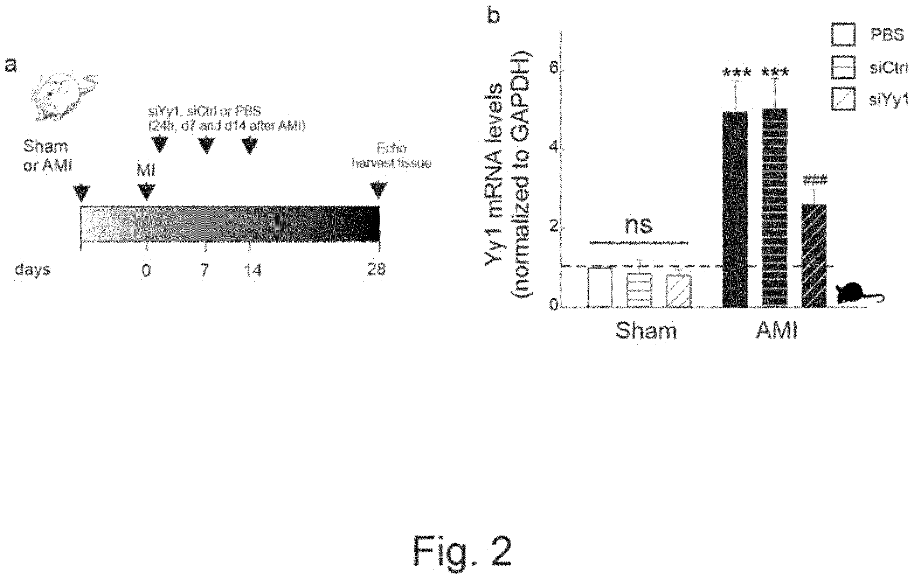

**Figure 2****.-** siYy1 therapy prevents the up-regulation of cardiac Yy1 transcription factor in infarcted LV area. (a) Representative scheme experimental procedure. (b) Yy1 mRNA levels; data were normalized respect to GAPDH mRNA levels. *** p<0.001 respect to their respective sham groups; ### p<0.001 respect to AMI+siCtrl group. siCtrl: interference RNA control; siYy1: specific interference ARN against Yy1 transcription factor; AMI: acute myocardial infarction; ns: non-significant; Yy1: Yin yang-1 transcription factor.

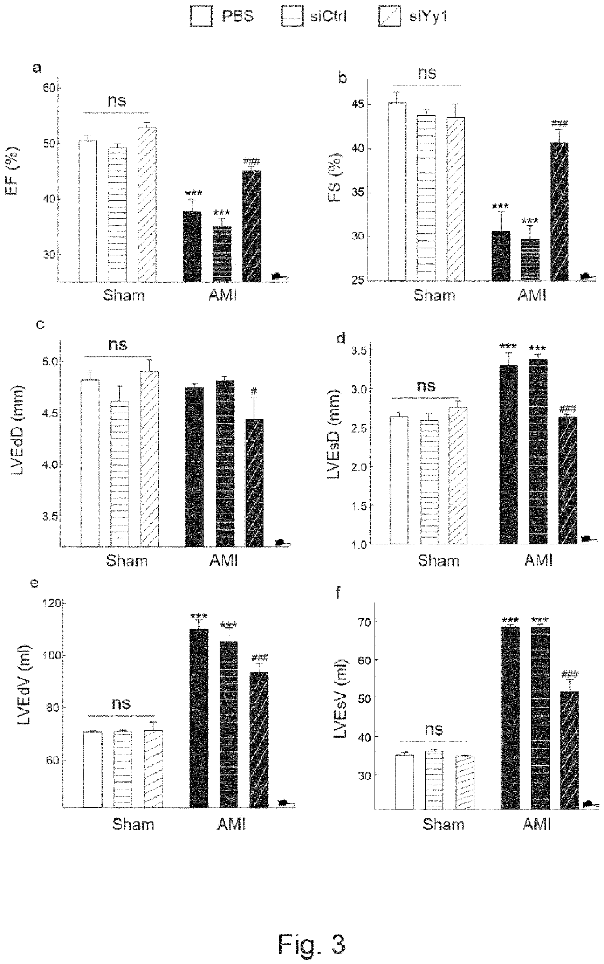

**Figure 3****.-** siYy1 therapy improves cardiac function following AMI. (a-f) Echocardiographic determined EF (a), FS (b), LVEDD (c), LVEsD (d), LVEdV (e), or LVEsV (f), respectively. *p<0.05, *** p<0.001 respect to their respective sham groups; ### p<0.001 respect to AMI+siCtrl group. EF: ejection fraction; FS: fractional shorting; LVEdD: left ventricular end diastolic dimension; LVEsD: left ventricular end systolic dimension; LVEdV: Left ventricular end diastolic volume; LVEsV: Left ventricular end systolic volume; other abbreviations are shown above.

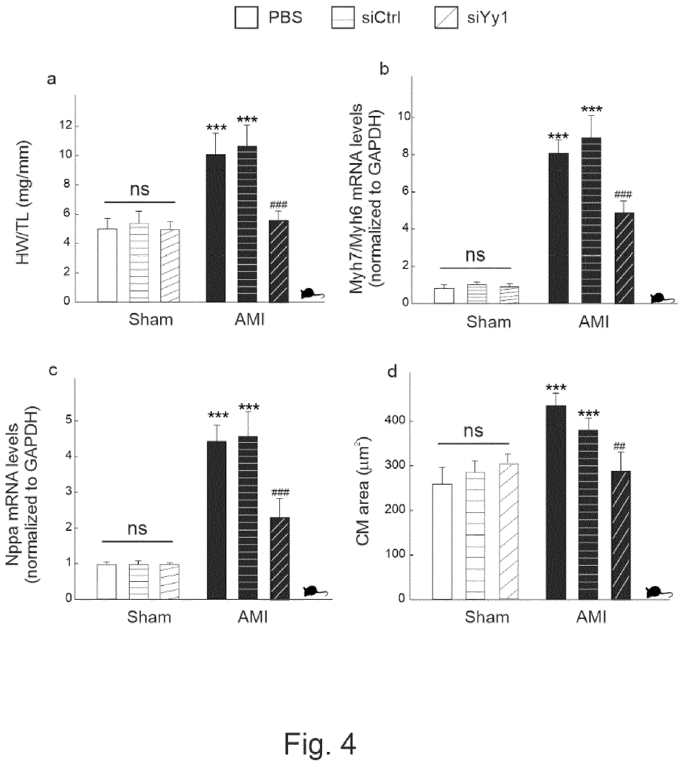

**Figure 4****.-** siYy1 therapy improves cardiac function and AMI-induced cardiac hypertrophy, (a) Ratio between heart weight and tibia length (HW/TL) as a measure of cardiac hypertrophy, (b, c) Real-time PCR quantification of markers for cardiac remodeling in left ventricular tissue; Myh7/Myh6, ratio of mRNAs encoding β- and α-myosin heavy chain. Nppa, atrial natriuretic peptide. (d) CM area. ns: non-significative; *** p < 0.001 respect to their respective sham group. ## p<0.01, ### p<0.001, respect to AMI+siCtrl group. CM: cardiomyocyte.

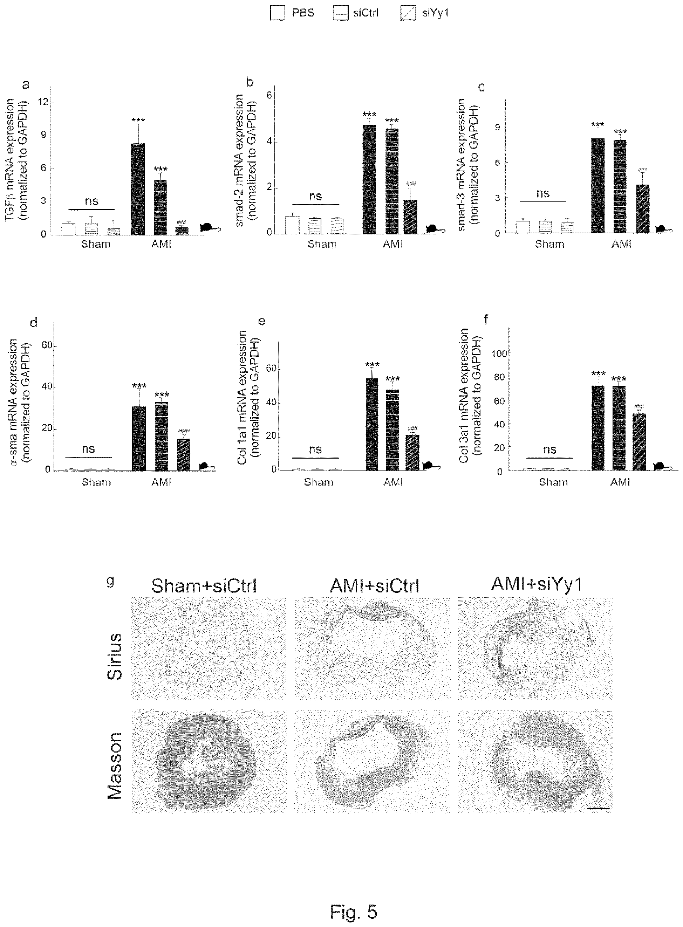

**Figure 5****.-** siYy1 therapy prevents cardiac fibrosis following AMI. (a-f) TGF-β, smad-2, smad-3, α-sma, col1a1, and col3a1 mRNA levels; data were normalized to GAPDH mRNA levels. (g) Representative image sections from Sirius Red and Masson-stained myocardium of the indicated groups; Scale bar: 0.25 cm. ns: non-significative; *** p < 0.001 respect to their respective sham group. ### p<0.001, respect to AMI+siCtrl group.

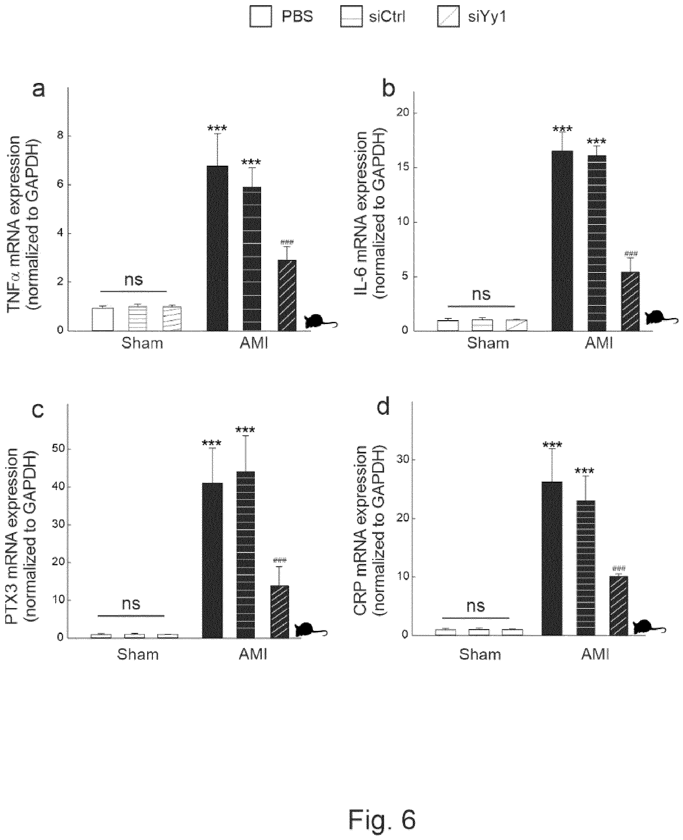

**Figure 6****.-** siYy1 therapy blocks adverse cardiac inflammation following AMI. (a-d) TNFα, IL-6, PTX3 and CRP mRNA levels; data were normalized to GAPDH mRNA levels. ns: non-significative; *** p < 0.001 respect to their respective sham group; ### p<0.001, respect to AMI+siCtrl group. TNF: Tumoral necrosis factor; IL: interleukin; PTX: pentraxin: CRP: C-reactive protein.

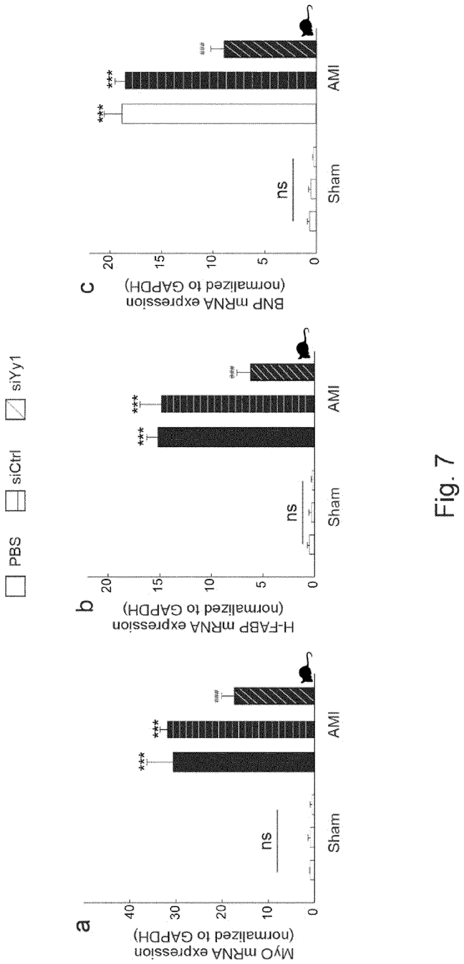

**Figure 7****.-** siYy1 therapy improves myocardial death following AMI. (a-c) MyO, H-FABP and BNP mRNA levels; data are normalized to GAPDH mRNA levels. *** p < 0.001 respect to their respective sham group; ### p<0.001, respect to AMI+siCtrl group. MyO: myoglobin; H-FABP: Fatty acid-binding protein; BNP: brain natriuretic peptide.

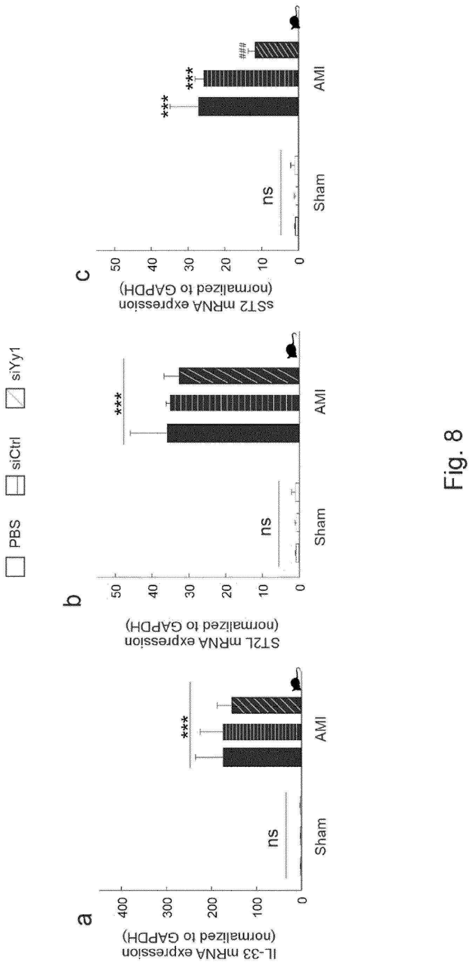

**Figure 8****.-** siYy1 therapy modulates IL-33/ST2L axis. (a-c) IL-33, ST2L and sST2 mRNA levels; data are normalized respect to GAPDH mRNA levels. *** p<0.001 respect to their respective controls; ### p<0.001 respect to AMI+siCtrl group. IL: interleukin; ST2L: membrane receptor ST2L; sST2: soluble isoform ST2.

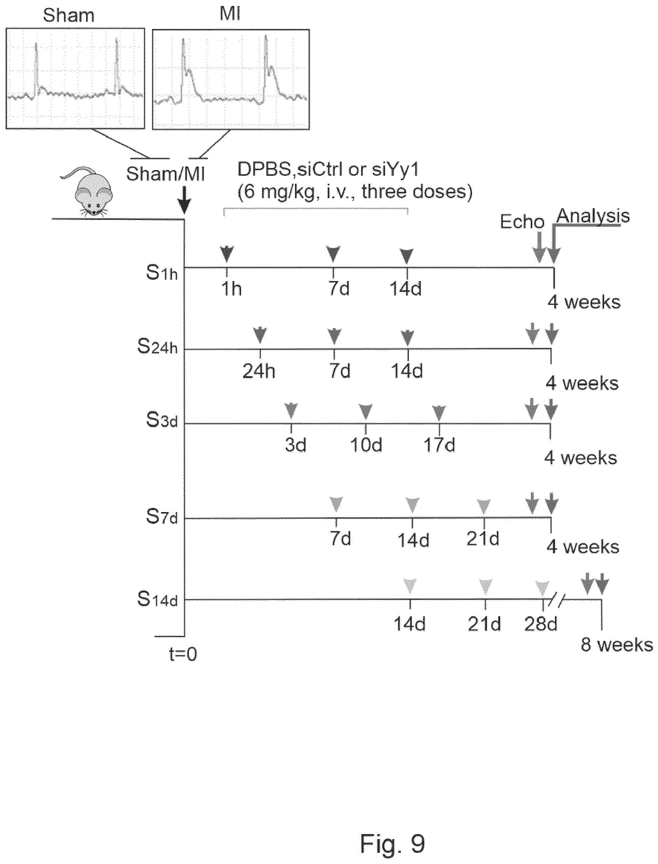

**Figure 9****.** Representative experimental design of time-dependent strategies. Design of the study based on different times of siYy1 initiation post-MI. Black arrows indicate the sham/MI procedures. Arrow heads indicate the temporal points of treatment. At 30 min post-MI, survival animals were randomized into two global groups: MI and Sham. Animals were randomized to different treatment subgroups according to initiation times of siYy1 therapy, or siCtrl or DPBS, after MI: 1 hour (=S1h), 24 hours (=S24h), 3 days (=S3d), 7 days (=S7d) or 14 days (=S14d). SiYy1 therapy (6 mg/kg i.v.) was repeated every 7 days until completing 3 doses Animals in the subgroups S1h, S24h, S3d and S7d were sacrificed at 4 weeks post-MI, while those in S14d were sacrificed at 8 weeks after MI.

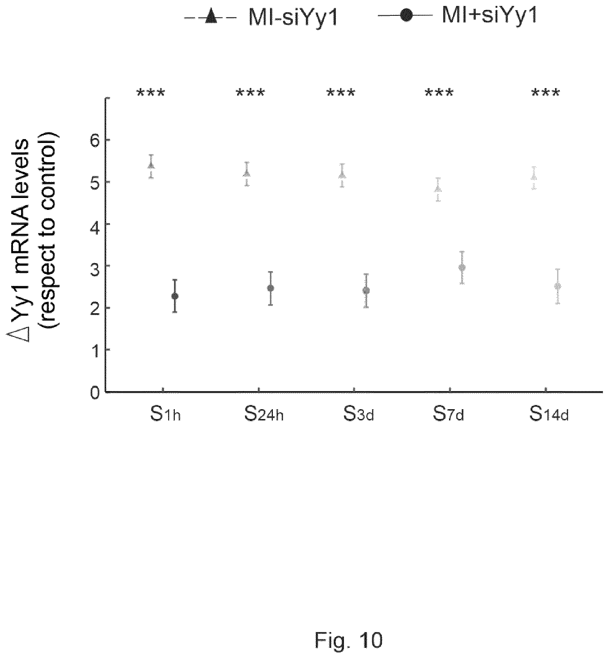

**Figure 10****.** SiYy1 therapy induced a down-regulation of Yy1 transcription factor after MI regardless of time of initiation. Yy1 mRNA levels in the infarcted LV region 4 weeks after MI (from S1h to S7d groups) or 8 weeks after MI (S14d group). Compared to sham groups, the Yy1 mRNA levels in the infarcted LV myocardium were elevated in presence of MI (MI-siYy1) and were significantly reduced by siYy1 therapy in all treatment groups (MI+siYy1) (p<0.001, in all cases).

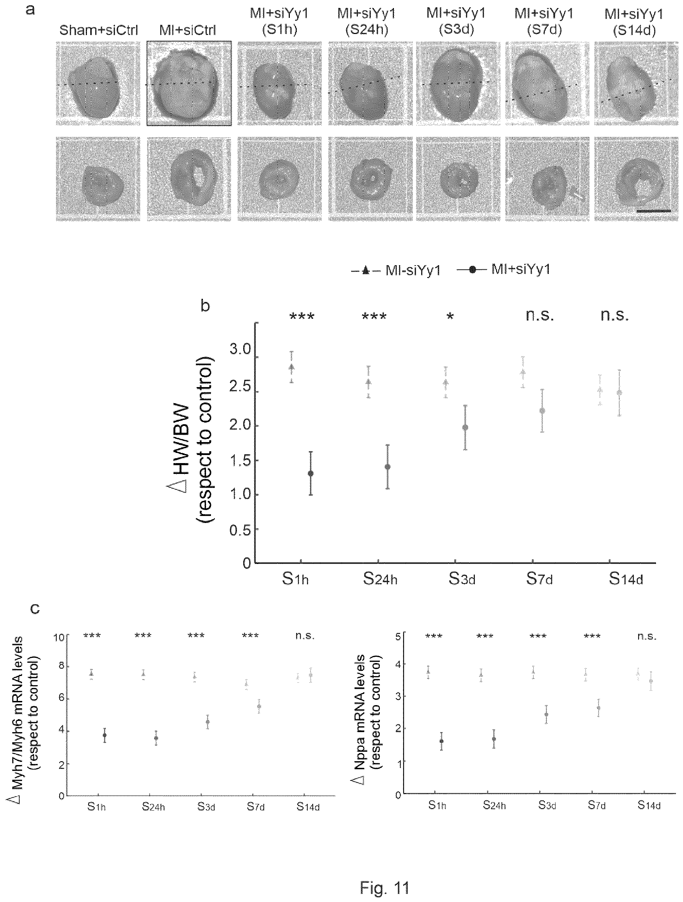

**Figure 11****.** siYy1 therapy protects against cardiac hypertrophy after MI. (a) Representative images of hearts harvested 4 weeks (S1h to S7d) or 8 weeks (S14d) post-MI and treated, or not, with siYy1; Scale bar: 0.5 cm. (b) Heart weight-to-body weight ratio (c) Quantitative real-time PCR analysis of molecular markers for cardiac myocyte hypertrophy (Myh7/Myh6 and Nppa). PCR performed with 2 replicates each. All quantitative data are reported as means ± SEM of changes between MI and sham (MI - Sham). Calculated means were marginal means to correct for the different number of animals in each group. Contrasts were performed to compare the differences in changes (diff-in-diffs) between treatments according to each start (Student's t-tests). While a dashed line (triangle) indicates a non-therapy MI, continuous line (circle) indicates a MI treated with siYy1. *** p < 0.001, **p<0.01 and *p<0.05. Abbreviations: BW: body weight; DPBS: balanced salt solution; HW: heart weight; LW: left ventricle weight; Myh: myosin heavy chain; Nppa: natriuretic Peptide A; ns: non-significance; S1h... S14d: start of treatment; siCtrl: interference RNAs control; siYy1: specific interference RNAs composition to silencing Yy1. Others abbreviations as before. siYy1 therapy initiated within the first 3 days protected from MI-induced cardiac hypertrophy, in terms of macroscopic cardiac hypertrophy (a), HWBW ratio (b). siYy1 therapy initiated within the first 7 days protected from MI-induced cardiac hypertrophy, in terms of levels of hypertrophic-associated marker genes Myh7 (related to Myh6) and Nppa (c). siYy1 therapy initiated at 14 days had not effect.

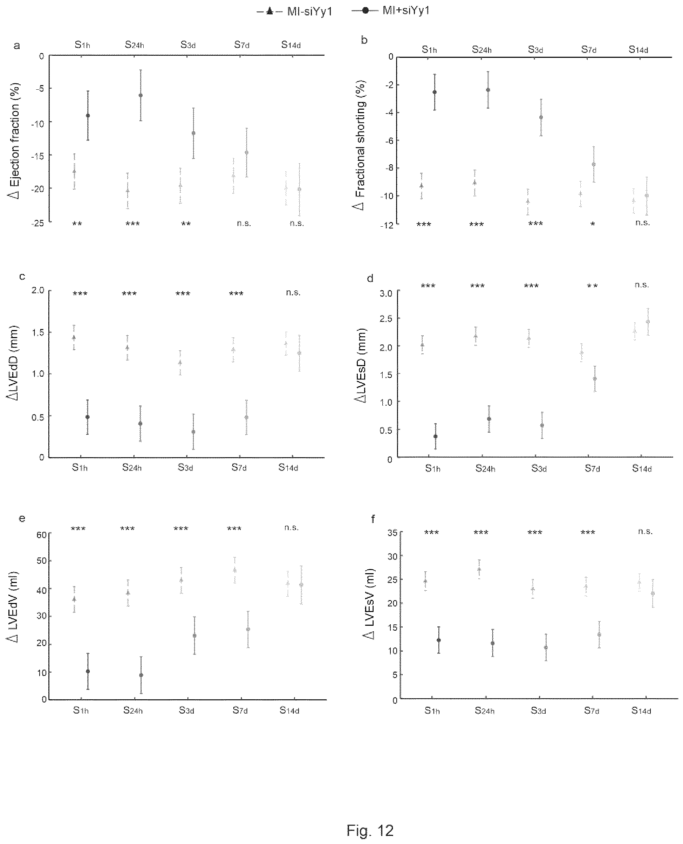

**Figure 12****.** siYy1 therapy protects against adverse LV remodeling after MI, assessed by echocardiography: systolic LV disfunction and LV dilatation. The RNA interference-based therapy to silence Yy1 protects cardiac dysfunction after MI. (a, b) Echocardiographic analysis of left ventricular ejection fraction (a) and left ventricular fractional shortening (b) in sham- and MI-operated mice 4 weeks after MI (from S1h to S7d groups) or 8 weeks after MI (S14d group). (c, d) Echocardiographic analysis of left ventricular end diastolic dimensions (c) and left ventricular end systolic dimensions (d) in sham- and MI-operated mice 4 weeks after MI (from S1h to S7d groups) or 8 weeks after MI (S14d group). (e, f) Echocardiographic analysis of left ventricular end diastolic volume (e) and left ventricular end systolic volume (f) in sham- and MI-operated mice 4 weeks after MI (from S1h to S7d groups) or 8 weeks after MI (S14d group). All quantitative data are reported as means ± SEM of changes between MI and sham (MI - Sham). Calculated means were marginal means to correct for the different number of animals in each group. Contrasts were performed to compare the differences in changes (diff-in-diffs) between treatments according to each start (Student's t-tests). While a dashed-line (triangle) indicates a non-therapy MI, continuous line (circle) indicates a MI treated with siYy1. ***p< 0.001, **p<0.01 and *p<0.05. Abbreviations: LVEdD: left ventricular end diastolic dimension; LVEsD: left ventricular end systolic dimension; LVEdV: left ventricular end diastolic volume; LVEsV: left ventricular end systolic volume; ml: milliliter; mm: millimeter. Others abbreviations as before. siYy1 therapy initiated within the first 3 days protected against LV systolic disfunction in terms of a significantly lower fall of LV ejection fraction (a) and fractional shortening (b) siYy1 therapy initiated within the first 7 days protected against LV enlargement in terms of a lower increase in LV end-diastolic and end-systolic diameters (c, d), and LV end-diastolic and end-systolic volumes (e, f). siYy1 therapy initiated at 14 days had not effect.

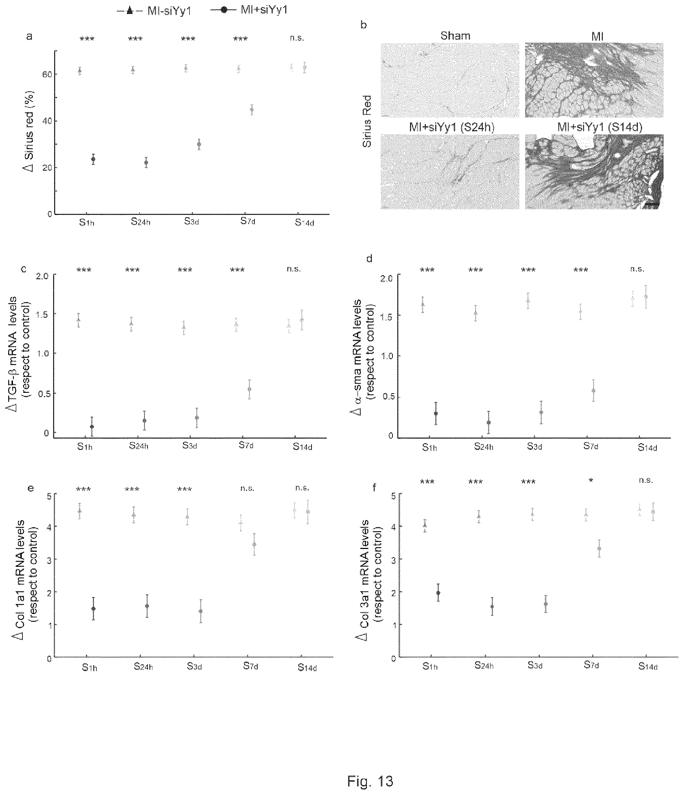

**Figure 13****.** siYy1 therapy prevents fibrosis following MI. siYy1 therapy initiated within the first 7 days protected against fibrosis in the infarcted myocardium, in terms of a significantly lower Sirius red staining (a) (representative images in panel b) as well as significantly lower levels of different fibrosis-associated marker genes deregulation (c-f). (a) Representative photomicrographs (×20) illustrating the Sirius Red staining in the indicated groups. Graph shows quantitative analysis of interstitial fibrosis. Scale bar: 100 µm. (b-e) Quantitative real-time PCR analysis of molecular markers for cardiac fibrosis (TGF-β, α-sma, col1a1, and col3a1). PCR performed with 2 replicates each. All quantitative data are reported as means ± SEM of changes between MI and sham (MI - Sham). Calculated means were marginal means to correct for the different number of animals in each group. Contrasts were performed to compare the differences in changes (diff-in-diffs) between treatments according to each start (Student's t-tests). While a dashed-line (triangle) indicates a non-therapy MI, continuous line (circle) indicates a MI treated with siYy1. ***p< 0.001 and *p<0.05. Abbreviations: α-sma: α-smooth muscle actin; TGF-β: Transforming growth factor beta; Col: Collagen. Others abbreviations as before. Therapy initiated at 14 days had not effect.

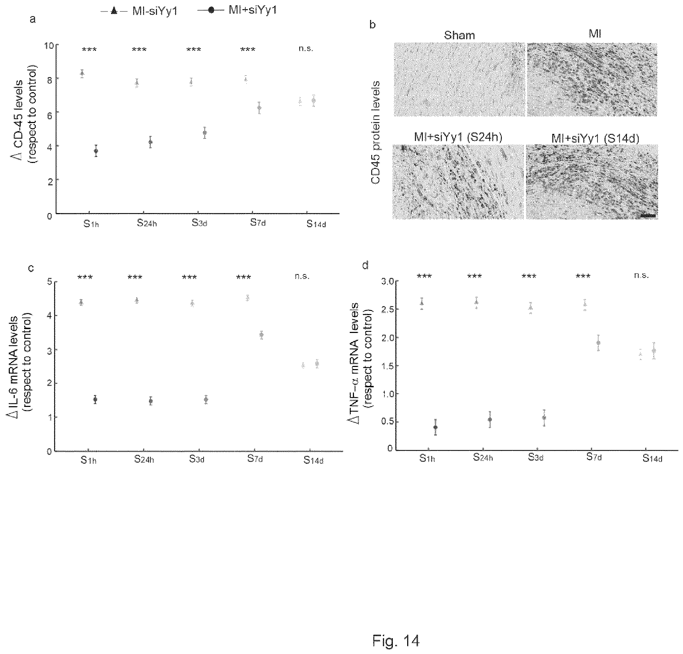

**Figure 14****.** siYy1 therapy protects against cardiac inflammation following MI. (a) Representative photomicrographs (×20) illustrating CD45 staining in the indicated groups. Graph shows quantitative analysis of CD45 positive. Scale bar: 100 µm. (b, c) Quantitative real-time PCR analysis of molecular markers for cardiac inflammation (IL-6 and TNFα). PCR performed with 2 replicates each. All quantitative data are reported as means ± SEM of changes between MI and sham (MI - Sham). Calculated means were marginal means to correct for the different number of animals in each group. Contrasts were performed to compare the differences in changes (diff-in-diffs) between treatments according to each start (Student's t-tests). While a dashed-line (triangle) indicates a non-therapy MI, continuous line (circle) indicates a MI treated with siYy1. ***p< 0.001. Abbreviations: IL: interleukin; TNF: Tumoral necrosis factor. Others abbreviations as before. siYy1 therapy initiated within the first 7 days protected against inflammation in the infarcted myocardium, in term of lower levels of CD45 positive staining (a) (representative images in panel b) and inflammation-associated specific markers (c-d). Therapy initiated at 14 days had not effect.

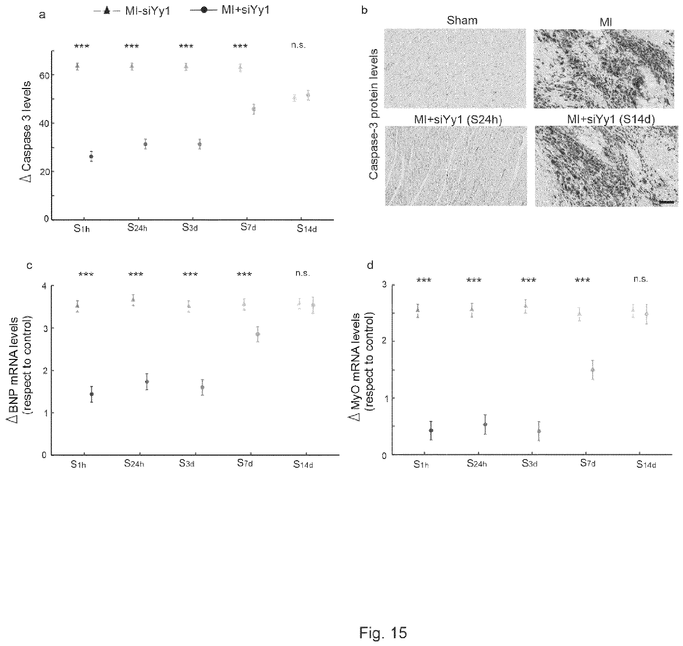

**Figure 15****.** siYy1 therapy protects against myocardial death following MI. (a) Representative photomicrographs (×20) illustrating active caspase 3 staining in the indicated groups. Graph shows quantitative analysis of active caspase 3. Scale bar: 100 µm. (b, c) Quantitative real-time PCR analysis of molecular markers for cardiac death (BNP and MyO). PCR performed with 2 replicates each. All quantitative data are reported as means ± SEM of changes between MI and sham (MI - Sham). Calculated means were marginal means to correct for the different number of animals in each group. Contrasts were performed to compare the differences in changes (diff-in-diffs) between treatments according to each start (Student's t-tests). While a dashed-line (triangle) indicates a non-therapy MI, continuous line (circle) indicates a MI treated with siYy1. ***p< 0.001. Abbreviations: MyO: myoglobin; H-FABP: Fatty acid-binding protein; BNP: brain natriuretic peptide. Others abbreviations as before. siYy1 therapy initiated within the first 7 days protected against cellular death in terms of a significantly lower increase of caspase 3 protein levels (a), as well as BNP and MyO mRNA levels (c-d).

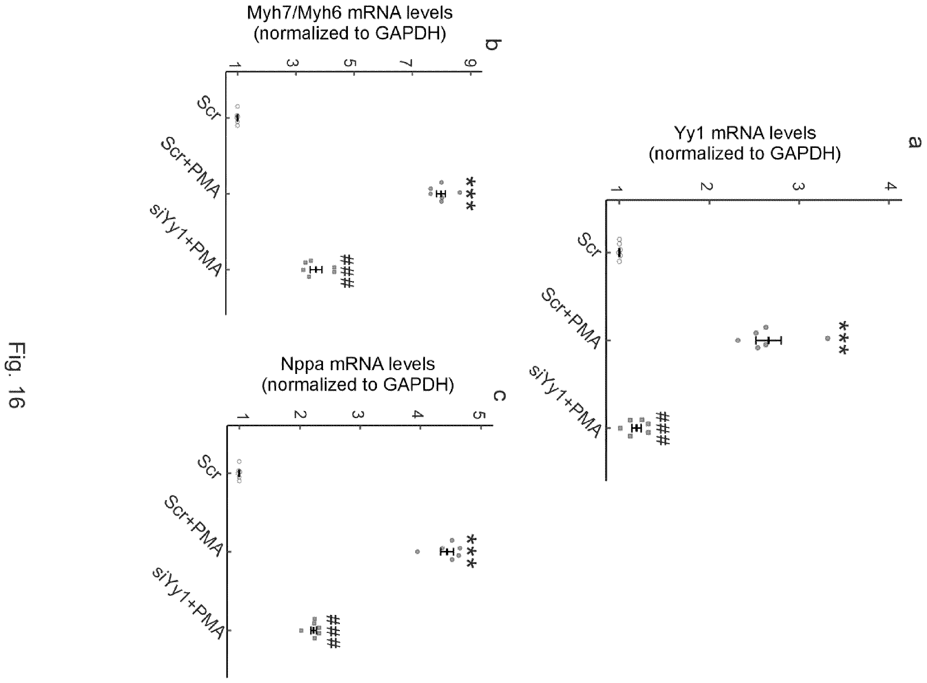

**Figure 16****.** siYy1 therapy prevents stretching-induced hypertrophy of human iPs-derived cardiomyocytes. (a) Quantification of Yy1 mRNA levels in CMs after stretching assessed. (b, c) Quantitative real-time PCR analysis of molecular markers for cardiac myocyte hypertrophy (Myh7/Myh6 and Nppa). PCR performed with 2 replicates each. All quantitative data are reported as means ± SEM. ***p< 0.001., compared to control group (Scr); ###p < 0.001, compared to Scr+PMA group; determined by two-way ANOVA followed by Bonferroni's post hoc test. Abbreviations: Scr: scramble. Others abbreviations as before.

### Brief description of the invention

An initial aspect of the invention refers to a composition comprising a compound capable of reducing the expression of the Yin Yang-1 (Yy1) gene in cardiac cells of a human or animal subject with respect to the expression observed in the absence of the compound in said cells, wherein the compound is an RNA interference (RNAi) of the Yy1 gene, and wherein said composition is for use in a method of treatment of left ventricular (LV) dysfunction following myocardial infarction (AMI) in the subject, and wherein said composition is administered between 12 hours and 7 days after the onset of myocardial infarction (AMI) in the subject.

Preferably, said composition is administered between 1 and 7 days after the onset of myocardial infarction (AMI) in the subject.

More preferably, said composition is administered between 12 hours and 3 days after the onset of myocardial infarction (AMI) in the subject. Still preferably, said composition is administered between 1 and 3 days after the onset of myocardial infarction (AMI) in the subject.

Still preferably, said composition is administered between 3 and 7 days after the onset of myocardial infarction (AMI) in the subject.

In a preferred embodiment, when the composition is administered between 12 hours and 3 days after the onset of myocardial infarction (AMI) in the subject, or administered between 1 and 3 days after the onset of myocardial infarction (AMI) in the subject, said composition is for use in a method of treatment of left ventricular (LV) dysfunction following myocardial infarction (AMI) in the subject by preventing, treating, mitigating, or reducing the loss of LV ejection fraction and/or fractional shortening and/or by preventing, treating, mitigating, or reducing MI-induced cardiac hypertrophy.

In a preferred embodiment, when the composition is administered between 1 and 7 days after the onset of myocardial infarction (AMI) in the subject, or administered between 3 and 7 days after the onset of myocardial infarction (AMI) in the subject, said composition is for use in a method of treatment of left ventricular (LV) dysfunction following myocardial infarction (AMI) in the subject by preventing, treating, mitigating, or reducing LV enlargement.

In a preferred embodiment, the interference RNA (RNAi) of the Yy1 gene is a siRNA selected from the list consisting of any of the following compounds: compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56.

In a preferred embodiment, the compound is administered intravenously.

In a preferred embodiment, the composition is a pharmaceutical composition comprising a therapeutically effective amount of an siRNA selected from the list consisting of any of the following compounds: compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56, or vectors which express these oligonucleotides and a pharmaceutically acceptable carrier.

### Description of the invention

The present invention relates generally to compounds which silence the endogenous Yy1 expression resulting in a decrease in the expression and release of sST2, which facilitates the cardioprotective response related to IL-33/ST2L axis; particularly to small interfering RNAs (siRNAs) that reduce Yy1 expression levels, and to the administration of these siRNAs in a subject in need thereof 2 to 48 hours after AMI, for the prevention or treatment of adverse myocardial remodeling, in particular in the prevention or treatment of cardiac complications following AMI such as myocardial fibrosis, cardiac inflammation, cardiac hypertrophy and/or functional impairment. Altogether, these findings solve the technical problem of providing an effective alternative treatment to reperfusion therapy two or more hours after the AMI.

Also, the present invention further generally provides compounds which silence the endogenous Yy1 expression resulting in a decrease in the expression and release of sST2, which facilitates the cardioprotective response related to IL-33/ST2L axis; particularly to small interfering RNAs (siRNAs) that reduce Yy1 expression levels, in an amount effective to down-regulate expression in a cell of the endogenous Yy1, in a method of therapy of left ventricular dysfunction, administered between 12 hours and 7 days after AMI. In particular, in a method of therapy initiated within the first 3 days after AMI to treat or protect against AMI-induced cardiac hypertrophy, preferably in terms of macroscopic cardiac hypertrophy. Preferably, in a method of therapy initiated within the first 7 days to treat or protect against AMI-induced cardiac hypertrophy, preferably in terms of levels of hypertrophic-associated marker genes Myh7 (related to Myh6) and Nppa. Preferably, in a method of therapy initiated within the first 3 days to treat or protect against LV systolic disfunction in terms of a significantly lower fall of LV ejection fraction and/or fractional shortening. Preferably, in a method of therapy initiated within the first 7 days after AMI to treat or protect against LV enlargement, preferably in terms of a lower increase in LV end-diastolic and end-systolic diameters and/or LV end-diastolic and end-systolic volumes.

It is herein noted that the Yy1 (Yin Yang-1) is a transcriptional repressor protein in humans that is encoded by the *Yy1* gene (Shi Y et al., Cell. 1991 Oct 18;67(2):377-88; Zhu W et al., Mamm Genome. 1994 Apr;5(4):234-6)
More preferably, the present invention relates to compounds which down-regulate the expression of the endogenous Yy1 in cardiac cells of a human or animal subject with respect to the expression observed in the absence of the compound in said cells, such as siRNAs, preferably those listed as compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56; and to the use of these compounds in the prevention or treatment of adverse myocardial remodeling following AMI, in particular in the prevention or treatment of cardiac complications following AMI such as myocardial fibrosis, cardiac inflammation, cardiac hypertrophy and/or functional impairment, wherein these compounds are administered to a subject in need thereof, 2 to 48 hours after the onset of the AMI. Preferably, such compounds are administered between 4 to 48 hours after AMI. More preferably, between 6 to 48 hours after AMI. Still more preferably, between 12 to 48 hours or between 24 to 48 hours after AMI. Still more preferably between 2, 4, 6 or 12 to 24 hours after AMI.

Also, the present invention refers to the use of the above-mentioned compounds in a method of therapy of left ventricular dysfunction, administered between 12 hours and 7 days after AMI, preferably between 1 and 7 days after AMI. In particular, in a method of therapy initiated within the first 3 days after AMI, preferably between 12 hours and 3 days, more preferably between 1 and 3 days, to treat or protect against AMI-induced cardiac hypertrophy, preferably in terms of macroscopic cardiac hypertrophy. Preferably, in a method of therapy initiated within the first 7 days, preferably between 12 hours and 7 days, more preferably between 1 and 7 days, to treat or protect against AMI-induced cardiac hypertrophy, preferably in terms of levels of hypertrophic-associated marker genes Myh7 (related to Myh6) and Nppa. Preferably, in a method of therapy initiated within the first 3 days, preferably between 12 hours and 3 days, more preferably between 1 and 3 days, to treat or protect against LV systolic disfunction in terms of a significantly lower fall of LV ejection fraction and/or fractional shortening. Preferably, in a method of therapy initiated within the first 7 days, preferably between 12 hours and 7 days, more preferably between 1 and 7 days, after AMI to treat or protect against LV enlargement, preferably in terms of a lower increase in LV end-diastolic and end-systolic diameters and/or LV end-diastolic and end-systolic volumes.

The invention further provides a use of a therapeutically effective dose of one or more compounds which down-regulate the expression of the endogenous Yy1, such as siRNAs, preferably those listed as compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56; for the preparation of a composition for promoting recovery in a patient suffering from adverse myocardial remodeling following AMI, in particular suffering from cardiac complications following AMI such as myocardial fibrosis, cardiac inflammation, cardiac hypertrophy and/or functional impairment, by administering said composition to a patient in need thereof within any of the time intervals indicated above after AMI.

The present invention thus provides methods and compositions for inhibiting expression of the endogenous Yy1 *in vivo* in cardiac cells of a human or animal subject with respect to the expression observed in the absence of the compound in said cells. In general, the method includes administering oligoribonucleotides, such as small interfering RNAs (i.e., siRNAs) that are targeted to the endogenous Yy1 in cardiac cells and hybridize to, or interact with, said mRNAs under biological conditions (within the cardiac cell), in an amount sufficient to down-regulate expression of the endogenous Yy1 by an RNA interference mechanism.

Thus, in accordance with the present invention, the siRNA molecules or inhibitors of the endogenous Yy1 may be used as drugs to prevent, treat or promote recovery in a patient in need thereof after an AMI, by administering these drugs within the time intervals indicated previously after the onset of the AMI. In particular, these drugs are administered to prevent or treat adverse myocardial remodeling following AMI, in particular to prevent or treat cardiac complications following AMI such as myocardial fibrosis, cardiac inflammation, cardiac hypertrophy and/or functional impairment. More particularly, the siRNA molecules or inhibitors of the endogenous Yy1 are used in a method of therapy of left ventricular dysfunction, administered between 12 hours and 7 days after AMI, preferably between 1 and 7 days after AMI. In particular, in a method of therapy initiated within the first 3 days after AMI, preferably between 12 hours and 3 days, more preferably between 1 and 3 days, to treat or protect against AMI-induced cardiac hypertrophy, preferably in terms of macroscopic cardiac hypertrophy. Preferably, in a method of therapy initiated within the first 7 days, preferably between 12 hours and 7 days, more preferably between 1 and 7 days, to treat or protect against AMI-induced cardiac hypertrophy, preferably in terms of levels of hypertrophic-associated marker genes Myh7 (related to Myh6) and Nppa. Preferably, in a method of therapy initiated within the first 3 days, preferably between 12 hours and 3 days, more preferably between 1 and 3 days, to treat or protect against LV systolic disfunction in terms of a significantly lower fall of LV ejection fraction and/or fractional shortening. Preferably, in a method of therapy initiated within the first 7 days, preferably between 12 hours and 7 days, more preferably between 1 and 7 days, after AMI to treat or protect against LV enlargement, preferably in terms of a lower increase in LV end-diastolic and end-systolic diameters and/or LV end-diastolic and end-systolic volumes.

The present invention thus provides double-stranded oligoribonucleotides (siRNAs), which down- regulate the expression of the endogenous Yy1 in cardiac cells of a human or animal subject with respect to the expression observed in the absence of the compound in said cells (from herein after **"Compound/s of the invention"** or **"siRNA of the invention").** An siRNA of the invention or a compound of the invention is a duplex oligoribonucleotide in which the sense strand is derived from the mRNA sequence of the endogenous Yy1, and the antisense strand is complementary to the sense strand. In general, some deviation from the target mRNA sequence is tolerated without compromising the siRNA activity (see e.g. Czauderna et al 2003 Nucleic Acids Research H(H), 2705-2716). An siRNA of the invention inhibits gene expression on a post-transcriptional level with or without destroying the mRNA. Without being bound by theory, siRNA may target the mRNA for specific cleavage and degradation and/ or may inhibit translation from the targeted message.

Generally, the siRNAs used in the present invention comprise a ribonucleic acid comprising a double stranded structure, whereby the double-stranded structure comprises a first strand and a second strand, whereby the first stand comprises a first stretch of contiguous nucleotides and whereby said first stretch is at least partially complementary to a target nucleic acid (the endogenous Yy1), and the second strand comprises a second stretch of contiguous nucleotides and whereby said second stretch is at least partially identical to a target nucleic acid (the endogenous Yy1). The strands may be modified on the sugar and /or on the phosphate and /or on the base, or alternatively may be unmodified. In one embodiment of the invention the said first strand and/or said second strand comprises a plurality of groups of modified nucleotides having a modification at the 2'-position whereby within the strand each group of modified nucleotides is flanked on one or both sides by a flanking group of nucleotides whereby the flanking nucleotides forming the flanking group of nucleotides is either an unmodified nucleotide or a nucleotide having a modification different from the modification of the modified nucleotides. Further, said first strand and/or said second strand may comprise said plurality of modified nucleotides and may comprises said plurality of groups of modified nucleotides.

The group of modified nucleotides and/or the group of flanking nucleotides may comprise a number of nucleotides whereby the number is selected from the group comprising one nucleotide to 10 nucleotides. In connection with any ranges specified herein it is to be understood that each range discloses any individual integer between the respective figures used to define the range including said two figures defining said range. In the present case the group thus comprises one nucleotide, two nucleotides, three nucleotides, four nucleotides, five nucleotides, six nucleotides, seven nucleotides, eight nucleotides, nine nucleotides and ten nucleotides.

The pattern of modified nucleotides of said first strand may be shifted by one or more nucleotides relative to the pattern of modified nucleotides of the second strand.

The modifications discussed above may be selected from the group comprising amino, fluoro, methoxy alkoxy, alkyl, amino, fluoro, chloro, bromo, CN, CF, imidazole, caboxylate, thioate, Ci to Cio lower alkyl, substituted lower alkyl, alkaryl or aralkyl, OCF3, OCN, O-, S-, or N- alkyl; O-, S-, or N-alkenyl; SOCH3; SO2CH3; ONO2; NO2, N3; heterozycloalkyl; heterozycloalkaryl; aminoalkylamino; polyalkylamino or substituted silyl, as, among others, described in European patents EP O 586 520 B1 or EP O 618 925 B1.

The double stranded structure of the siRNA may be blunt ended, on one or both sides. More specifically, the double stranded structure may be blunt ended on the double stranded structure's side which is defined by the 5'- end of the first strand and the 3'-end of the second strand, or the double stranded structure may be blunt ended on the double stranded structure's side which is defined by at the 3'-end of the first strand and the 5'~end of the second strand.

Additionally, at least one of the two strands may have an overhang of at least one nucleotide at the 5'- end; the overhang may consist of at least one deoxyribonucleotide. At least one of the strands may also optionally have an overhang of at least one nucleotide at the 3'-end. The length of the double-stranded structure of the siRNA is typically from about 17 to 27 and more preferably 19 or 21 bases. Further, the length of said first strand and/or the length of said second strand may independently from each other be selected from the group comprising the ranges of from about 15 to about 27 bases, 17 to 21 bases and 18 or 19 bases. A particular example is 27 bases. Additionally, the complementarity between said first strand and the target nucleic acid may be perfect, or the duplex formed between the first strand and the target nucleic acid may comprise at least 15 nucleotides wherein there is one mismatch or two mismatches between said first strand and the target nucleic acid forming said double-stranded structure.

In some cases both the first strand and the second strand each comprise at least one group of modified nucleotides and at least one flanking group of nucleotides, whereby each group of modified nucleotides comprises at least one nucleotide and whereby each flanking group of nucleotides comprising at least one nucleotide with each group of modified nucleotides of the first strand being aligned with a flanking group of nucleotides on the second strand, whereby the most terminal 5' nucleotide of the first strand is a nucleotide of the group of modified nucleotides, and the most terminal 3' nucleotide of the second strand is a nucleotide of the flanking group of nucleotides. Each group of modified nucleotides may consist of a single nucleotide and/or each flanking group of nucleotides may consist of a single nucleotide.

Additionally, it is possible that on the first strand the nucleotide forming the flanking group of nucleotides is an unmodified nucleotide which is arranged in a 3' direction relative to the nucleotide forming the group of modified nucleotides, and on the second strand the nucleotide forming the group of modified nucleotides is a modified nucleotide which is arranged in 5' direction relative to the nucleotide forming the flanking group of nucleotides.

Further the first strand of the siRNA may comprise eight to twelve, preferably nine to eleven, groups of modified nucleotides, and the second strand may comprise seven to eleven, preferably eight to ten, groups of modified nucleotides.

The first strand and the second strand may be linked by a loop structure, which may be comprised of a non-nucleic acid polymer such as, inter alia, polyethylene glycol. Alternatively, the loop structure may be comprised of a nucleic acid.

Further, the 5'-terminus of the first stand of the siRNA may be linked to the 3'-terminus of the second strand, or the 3'-end of the first stand may be linked to the 5'-terminus of the second strand, said linkage being via a nucleic acid linker typically having a length between 10-2000 nucleobases.

In particular, and as already indicated previously, the invention provides a compound of the invention or an siRNA of the invention selected from the group consisting of compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56, wherein any of these compounds may optionally include any of the modifications indicated throughout the present description. Preferably, the invention provides a compound of the invention or an siRNA of the invention selected from the group consisting of compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56, wherein any of these compounds may optionally include any of the modifications indicated throughout the present description.

It will be thus readily understood by those skilled in the art that the compounds of the present invention consist of a plurality of nucleotides, which are linked through covalent linkages. Each such covalent linkage may be a phosphodiester linkage, a phosphothioate linkage, or a combination of both, along the length of the nucleotide sequence of the individual strand. Other possible backbone modifications are described inter alia in U.S. Patent Nos. 5,587,361; 6,242,589; 6,277,967; 6,326,358; 5,399,676; 5,489,677; and 5,596,086.

The invention further provides a vector capable of expressing any of the aforementioned oligoribonucleotides in unmodified form in a cell after which appropriate modification may be made.

The invention also provides a composition comprising one or more of the compounds of the invention or siRNAs of the invention in a carrier, preferably a pharmaceutically acceptable carrier. This composition may comprise a mixture of two or more different siRNAs.

More particular, the invention provides a composition comprising a carrier and one or more of the compounds of the invention in an amount effective to down-regulate expression in a cell of the endogenous Yy1. In particular, and as already indicated and as explained in example 2, the experimental data shown in figure 11 indicates that siYy1 therapy, with any composition comprising a carrier and one or more of the compounds of the invention in an amount effective to down-regulate expression in a cell of the endogenous Yy1, initiated within the first 3 days protected from MI-induced cardiac hypertrophy, in terms of macroscopic cardiac hypertrophy (a), HWBW ratio (b). siYy1 therapy initiated within the first 7 days protected from MI-induced cardiac hypertrophy, in terms of levels of hypertrophic-associated marker genes Myh7 (related to Myh6) and Nppa (c). siYy1 therapy initiated at 14 days had not effect. Figure 12 shows that siYy1 therapy initiated within the first 3 days protected against LV systolic disfunction in terms of a significantly lower fall of LV ejection fraction (a) and fractional shortening (b) siYy1 therapy initiated within the first 7 days protected against LV enlargement in terms of a lower increase in LV end-diastolic and end-systolic diameters (c, d), and LV end-diastolic and end-systolic volumes (e, f). siYy1 therapy initiated at 14 days had not effect. Figure 13 shows that siYy1 therapy initiated within the first 7 days protected against fibrosis in the infarcted myocardium, in terms of a significantly lower Sirius red staining (a) (representative images in panel b) as well as significantly lower levels of different fibrosis-associated marker genes deregulation (TGF-β, α-sma, Col1a1 and Col3a1 (c-f). Therapy initiated at 14 days had not effect. Figure 14 shows that siYy1 therapy initiated within the first 7 days protected against inflammation in the infarcted myocardium, in term of lower levels of CD45 positive staining (a) (representative images in panel b) and inflammation-associated specific markers (c-d). Therapy initiated at 14 days had not effect. Figure 15 shows that siYy1 therapy initiated within the first 7 days protected against cellular death in terms of a significantly lower increase of caspase 3 protein levels (a), as well as BNP and MyO mRNA levels (c-d). Figure 16 shows that siYy1 therapy prevents stretching-induced hypertrophy of human iPs-derived cardiomyocytes. Compared to control groups (Scr), the Yy1 mRNA levels in human iPs-derived cardiomyocytes (hiPsCMs) were elevated after stretching (PMA+Scr) (p<0.001) and were significantly reduced by siYy1 therapy (siYy1+PMA) (p<0.001) (a). Moreover, siYy1 therapy protected from stretching-induced cardiac hypertrophy, in terms of levels of hypertrophic-associated marker genes Myh7 (related to Myh6) and Nppa (b, c).

The present invention thus provides a method of treatment of left ventricular (LV) dysfunction following an acute myocardial infarction, between 12 hours and 7 days after AMI in a patient, comprising administering to the patient a compound or composition described in the invention in a therapeutically effective dose so as to thereby prophylactically or therapeutically treat the patient. Preferably, the method is administered between 1 and 7 days after AMI. More preferably, between 12 hours and 3 days after AMI, preferably between 1 and 3 days after AMI. Also preferably, between 3 and 7 days after AMI.

The present invention further provides a method of treatment of adverse myocardial remodeling following AMI, in particular in the prevention or treatment of cardiac complications following AMI such as myocardial fibrosis, cardiac inflammation, cardiac hypertrophy and/or functional impairment, more particularly in a method of treatment of left ventricular dysfunction following an acute myocardial infarction, 2 to 48 hours after AMI in a patient, comprising administering to the patient a compound or composition described in the invention in a therapeutically effective dose so as to thereby prophylactically or therapeutically treat the patient. Preferably, the method is administered between 4 to 48 hours after AMI. More preferably, between 6 to 48 hours after AMI. Still more preferably, between 12 to 48 hours or 24 to 48 hours after AMI. Still more preferably, between 2, 4, 6 or 12 to 24 hours after AMI.

The present invention further provides a method of treatment of left ventricular (LV) dysfunction following an acute myocardial infarction, 12 hours to 7 days after AMI in a patient, comprising administering to the patient a compound or composition described in the invention in a therapeutically effective dose so as to thereby prophylactically or therapeutically treat the patient. Preferably, the method is administered between 1 and 7 days after AMI. More preferably, between 12 hours and 3 days after AMI, preferably between 1 and 3 days after AMI. Still more preferably, between 3 and 7 days after AMI.

In a preferred embodiment, the present invention provides a method of treatment of left ventricular (LV) dysfunction following an acute myocardial infarction, preferably by preventing, treating, mitigating, or reducing the loss of LV ejection fraction and/or fractional shortening, 12 to 72 hours after AMI in a patient, comprising administering to the patient a compound or composition described in the invention in a therapeutically effective dose so as to thereby prophylactically or therapeutically treat the patient. Preferably, the method is administered between 24 to 72 hours after AMI. More preferably, between 36 to 72 hours after AMI. Still more preferably, between 48 to 72 hours after AMI.

In a preferred embodiment, the present invention provides a method of treatment of left ventricular (LV) dysfunction following an acute myocardial infarction, preferably by preventing, treating, mitigating, or reducing MI-induced cardiac hypertrophy, preferably in terms of macroscopic cardiac hypertrophy, 12 to 72 hours after AMI in a patient, comprising administering to the patient a compound or composition described in the invention in a therapeutically effective dose so as to thereby prophylactically or therapeutically treat the patient. Preferably, the method is administered between 24 to 72 hours after AMI. More preferably, between 36 to 72 hours after AMI. Still more preferably, between 48 to 72 hours after AMI.

In a preferred embodiment, the present invention provides a method of treatment of left ventricular (LV) dysfunction following an acute myocardial infarction, preferably by preventing, treating, mitigating, or reducing the LV enlargement, preferably in terms of a lower increase in LV end-diastolic and end-systolic diameters and/or LV end-diastolic and end-systolic volumes, 3 to 7 days after AMI after AMI in a patient, comprising administering to the patient a compound or composition described in the invention in a therapeutically effective dose so as to thereby prophylactically or therapeutically treat the patient.

Delivery: Delivery systems aimed specifically at the enhanced and improved delivery of siRNA into mammalian cells have been developed, see, for example, Shen et al (FEBS letters 539: 111-114 (2003)), Xia et al., Nature Biotechnology 20: 1006-1010 (2002), Reich et al., Molecular Vision 9: 210-216 (2003), Sorensen et al. (J.Mol.Biol. 327: 761-766 (2003), Lewis et al., Nature Genetics 32: 107-108 (2002) and Simeoni et al., Nucleic Acids Research 31, 11 : 2717-2724 (2003). siRNA have been successfully used for inhibition in primates; for further details see Tolentino et al., Retina 24(1) February 2004 I 132-138. Respirator}' formulations for siRNA are described in U.S. patent application No. 2004/0063654 of Davis et al. Cholesterol-conjugated siRNAs (and other steroid and lipid conjugated siRNAs) can been used for delivery see Soutschek et al Nature 432: 173- 177(2004). Therapeutic silencing of an endogenous gene by systemic administration of modified siRNAs; and Lorenz et al. Bioorg. Med. Chemistry. Lett. 14:4975-4977 (2004) Steroid and lipid conjugates of siRNAs to enhance cellular uptake and gene silencing in liver cells.

The compounds, siRNAs or pharmaceutical compositions of the present invention are administered and dosed in accordance with good medical practice, taking into account the clinical condition of the individual patient, the site and method of administration, scheduling of administration, patient age, sex, body weight and other factors known to medical practitioners.

The "therapeutically effective dose" for purposes herein is thus determined by such considerations as are known in the art. The dose must be effective to achieve improvement including but not limited to improved survival rate or more rapid recovery, or improvement or elimination of symptoms and other indicators as are selected as appropriate measures by those skilled in the art. The compounds of the present invention can be administered by any of the conventional routes of administration. It should be noted that the compound can be administered as the compound or as pharmaceutically acceptable salt and can be administered alone or as an active ingredient in combination with pharmaceutically acceptable carriers, solvents, diluents, excipients, adjuvants and vehicles. The compounds can be administered orally, subcutaneously or parenterally including intravenous, intraarterial, intramuscular, intraperitoneally, and intranasal administration as well as intrathecal and infusion techniques. Preferably, the compounds, siRNAs or pharmaceutical compositions of the invention are administered parenterally, preferably by the intravenous route.

Implants of the compounds are also useful. Liquid forms may be prepared for injection, the term including subcutaneous, transdermal, intravenous, intramuscular, intrathecal, and other parental routes of administration. The liquid compositions include aqueous solutions, with and without organic co-solvents, aqueous or oil suspensions, emulsions with edible oils, as well as similar pharmaceutical vehicles. In addition, under certain circumstances the compositions for use in the novel treatments of the present invention may be formed as aerosols, for intranasal and like administration. The patient being treated is a warm-blooded animal and, in particular, mammals including man. The pharmaceutically acceptable carriers, solvents, diluents, excipients, adjuvants and vehicles as well as implant earners generally refer to inert, non-toxic solid or liquid fillers, diluents or encapsulating material not reacting with the active ingredients of the invention and they include liposomes and microspheres. Examples of delivery systems useful in the present invention include U. S. Patent Nos. 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art. In one specific embodiment of this invention topical and transdermal formulations are particularly preferred.

In general, the active dose of compound for humans is in the range of from l ng/kg to about 20-100 mg/kg body weight per day, preferably about 0.01 mg to about 2-10 mg/kg body weight per day.

The term "treatment" as used herein refers to administration of a therapeutic substance effective to ameliorate symptoms associated with a disease, to lessen the severity or cure the disease, or to prevent the disease from occurring. In a particular embodiment, the administration comprises intravenous administration. In another particular embodiment the administration comprises topical or local administration.

Another aspect of the invention is a method of treatment in a patient of myocardial fibrosis, cardiac inflammation, cardiac hypertrophy and/or functional impairment within the time intervals indicated throughout the present specification after AMI, comprising administering to the patient a pharmaceutical composition of the invention in a therapeutically effective amount so as to thereby prophylactically or therapeutically treat the patient.

In another aspect of the invention a pharmaceutical composition is provided which comprises any of compounds 1 to 3 or vectors which express these oligonucleotides and a pharmaceutically acceptable carrier. Another aspect of the invention is the use of a therapeutically effective amount of any of the above oligoribonucleotides or vectors for the preparation of a medicament for promoting recovery in a patient suffering from myocardial fibrosis, cardiac inflammation, cardiac hypertrophy and/or functional impairment within the time intervals indicated throughout the present specification after AMI.

The present invention also provides for a process of preparing a pharmaceutical composition, which comprises admixing one or more compounds of the present invention with a pharmaceutically acceptable carrier.

In a preferred embodiment, the compound used in the preparation of a pharmaceutical composition is admixed with a carrier in a pharmaceutically effective dose. In a particular embodiment the compound of the present invention is conjugated to a steroid or to a lipid or to another suitable molecule e.g. to cholesterol.

Modifications or analogs of nucleotides can be introduced to improve the therapeutic properties of the nucleotides. Improved properties include increased nuclease resistance and/or increased ability to permeate cell membranes.

Accordingly, the present invention also includes all analogs of, or modifications to, a oligonucleotide of the invention that does not substantially affect the function of the polynucleotide or oligonucleotide. In a preferred embodiment such modification is related to the base moiety of the nucleotide, to the sugar moiety of the nucleotide and/or to the phosphate moiety of the nucleotide.

In embodiments of the invention, the nucleotides can be selected from naturally occurring or synthetically modified bases. Naturally occurring bases include adenine, guanine, cytosine, thymine and uracil. Modified bases of the oligonucleotides include inosine, xanthine, hypoxanthine, 2- aminoadenine, 6-methyl-, 2-propyl- and other alkyl- adenines, 5-halo uracil, 5-halo cytosine, 6-aza cytosine and 6-aza thymine, pseudo uracil, 4-thiuracil, 8-halo adenine, 8-aminoadenine, 8-thiol adenine, 8-thiolalkyl adenines, 8-hydroxyl adenine and other 8-substituted adenines, 8-halo guanines, 8-arnino guanine, 8-thiol guanine, 8-thioalkyl guanines, 8-hydroxyl guanine and other substituted guanines, other aza and deaza adenines, other aza and deaza guanines, 5-trifluoromethyl uracil and 5- trifluoro cytosine.

In addition, analogs of nucleotides can be prepared wherein the structures of the nucleotides are fundamentally altered and are better suited as therapeutic or experimental reagents. An example of a nucleotide analog is a peptide nucleic acid (PNA) wherein the deoxyribose (or ribose) phosphate backbone in DNA (or RNA) is replaced with a polyamide backbone similar to that found in peptides. PNA analogs have been shown to be resistant to degradation by enzymes and to have extended lives *in vivo* and *in vitro.* Further, PNAs have been shown to bind more strongly to a complementary DNA sequence than to a DNA molecule. This observation is attributed to the lack of charge repulsion between the PNA strand and the DNA strand. Other modifications that can be made to oligonucleotides include polymer backbones, cyclic backbones, or acyclic backbones.

In one embodiment the modification is a modification of the phosphate moiety, whereby the modified phosphate moiety is selected from the group comprising phosphothioate.

The compounds of the present invention can be synthesized by any of the methods that are well-known in the art for synthesis of ribonucleic (or deoxyribonucleic) oligonucleotides. Such synthesis is, among others, described in Beaucage S.L. and Iyer R.P., Tetrahedron 1992; 48: 2223-2311, Beaucage SX. and Iyer R.P., Tetrahedron 1993; 49: 6123-6194 and Caruthers M.H. et al., Methods Enzymol. 1987; 154: 287-313, the synthesis of thioates is, among others, described in Eckstein F., Annu. Rev. Biochem. 1985; 54: 367-402, the synthesis of RNA molecules is described in Sproat B., in Humana Press 2005 Edited by Herdewijn P.; Kap. 2: 17-31 and respective downstream processes are, among others, described in Pingoud A. et. al., in IRL Press 1989 Edited by Oliver R.W.A.; Kap. 7: 183-208 and Sproat B., in Humana Press 2005 Edited by Herdewijn P.; Kap. 2: 17-31 (supra).

Other synthetic procedures are known in the art e.g. the procedures as described in Usman et al., 1987, J. Am. Chem. Soc, 109, 7845; Scaringe et al., 1990, Nucleic Acids Res., 18, 5433; Wincott et al., 1995, Nucleic Acids Res. 23, 2677-2684; and Wincott et al., 1997, Methods Mol. Bio., 74, 59, and these procedures may make use of common nucleic acid protecting and coupling groups, such as dimethoxytrityl at the 5'-end, and phosphoramidites at the 3 -end. The modified (e.g. 2'-O- methylated) nucleotides and unmodified nucleotides are incorporated as desired.

The oligonucleotides of the present invention can be synthesized separately and joined together post- synthetically, for example, by ligation (Moore et al., 1992, Science 256, 9923; Draper et al., International PCT publication No. WO93/23569; Shabarova et al., 1991, Nucleic Acids Research 19, 4247; Bellon et al., 1997, Nucleosides Sc Nucleotides, 16, 951; Bellon et al., 1997, Bioconjugate Chem. 8, 204), or by hybridization following synthesis and/or deprotection.

It is noted that a commercially available machine (available, inter alia, from Applied Biosystems) can be used; the oligonucleotides are prepared according to the sequences disclosed herein. Overlapping pairs of chemically synthesized fragments can be ligated using methods well known in the art (e.g., see U.S. Patent No. 6,121,426). The strands are synthesized separately and then are annealed to each other in the tube. Then, the double-stranded siRNAs are separated from the single-stranded oligonucleotides that were not annealed (e.g. because of the excess of one of them) by HPLC. In relation to the siRNAs or siRNA fragments of the present invention, two or more such sequences can be synthesized and linked together for use in the present invention.

The compounds of the invention can also be synthesized via a tandem synthesis methodology, as described in US patent application publication No. US2004/0019001 (McSwiggen), wherein both siRNA strands are synthesized as a single contiguous oligonucleotide fragment or strand separated by a cleavable linker which is subsequently cleaved to provide separate siRNA fragments or strands that hybridize and permit purification of the siRNA duplex. The linker can be a polynucleotide linker or a non-nucleotide linker.

The compounds of the present invention can be delivered either directly or with viral or non-viral vectors. When delivered directly the sequences are generally rendered nuclease resistant. Alternatively, the sequences can be incorporated into expression cassettes or constructs such that the sequence is expressed in the cell as discussed herein below. Generally, the construct contains the proper regulatory sequence or promoter to allow the sequence to be expressed in the targeted cell. Vectors optionally used for delivery of the compounds of the present invention are commercially available, and may be modified for the purpose of delivery of the compounds of the present invention by methods known to one of skill in the art.

The following examples illustrate but do not limit the present invention.

### Examples

### Example 1.

### MATERIAL AND METHODS

*Animal Care and ethical aspects.* C57B1/6J male mice (weighing 25-30 g) were purchased from the Jackson Laboratory. Mice were housed in specific pathogen free environment with a relative humidity of 50 ± 5% at 23 ± 2 °C with 12 h light and dark cycles. Mice had free access to food and water. All animal experiments were approved by the Ethics Review committee for animal use at the University of Murcia (Permit number: A13150105). After 7 days' adaptation, the animals were subjected to a surgical procedure to induce AMI before systemic Yy1 genetic silencing. Animals were randomly split into six groups (see groups and experimental design).

*Induction of experimental AMI in mice.* Before the surgical procedures, animals were anesthetized with intraperitoneal ketamine (75 mg/kg) and medetomidine (0.5 mg/kg), before being intubated and ventilated with an 18-gauge intravenous catheter and placed in supine position over a temperature control pad. The animals were monitored by electrocardiogram (ECG) electrodes connected to the limbs through small needles inserted subcutaneously. Left-sided thoracotomy was performed by a small incision between the third and fourth intercostal spaces. The incision was expanded by a blunt ended retractor in such a manner that the lungs were avoided in the area of retraction. The pericardial sac surrounding the heart was cut open, but the heart was not exteriorized. The ligation site of the left anterior descending coronary artery (LAD) was determined 4 mm away from the origin. Using a tapered atraumatic needle, a 8-0 silk ligature was passed underneath the LAD and tied with three knots. Visible blanching and cyanosis of the anterior wall of the left ventricle and swelling of the left atrium were taken as indicative of successful ligation. The procedure was considered successful if the ECG showed ST-segment elevation and the anterior wall of the left ventricle became blanched. Ribs and muscles were closed using 6-0 vicryl dissolvable sutures, leaving a small gap to aspirate any air left in the chest cavity. The air was aspirated by an in-house tube (2 mm diameter), again without touching the lungs. At the time of closure, neomycin powder and betadine were applied to the muscle and skin stitch sites, respectively. The surgical site was dressed daily to avoid any infection and to monitor for any dehiscence of the suture site. The entire procedure was performed within 20 min of the induction of anesthesia. Sham-operated rats underwent the same procedure without any ligation. After surgery, the animals received four doses of buprenorphine (0.05 mg/kg, subcutaneous) at 8 h intervals. The sham groups underwent the same surgical procedure except that the LAD coronary artery was not occluded. Electrocardiographic monitoring was used to confirm ST segment elevation after AMI induction. To evaluate the evolution of cardiac damage, an echocardiographic study was performed on each mouse before surgery, and after 24 h, 1 and 4 weeks following AMI(16).

*Experimental design and study protocol.* At 24 post-AMI, survived animals were randomize rinsed into six experimental groups: (1) Sham group treated with PBS by intravenous route (Sham group PBS, n=10); (2) Sham group treated with siControl (siCtrl, see table 3) by intravenous route (Sham groups siCtrl, n=10); (3) Sham group treated with siYy1 (the term "siYy1" shall be understood herein a a pool formed by four specific siRNA sequences to silent the endogenous Yy1 levels (compounds 1 to 4 of Table 3) by intravenous route (Sham group siYy1, n=10); (4) Infarcted group treated with placebo (PBS) by intravenous route (AMI group PBS, n=10); (5) Infracted group treated with siControl (siCtrl) by intravenous route (AMI group siCtrl, n=10); (6) Infracted group treated with siYy1 by intravenous route (AMI group siYy1, n=10) The animals were kept in the conditions described above until their sacrifice (4 weeks after AMI).

*Endogenous Yin-Yang 1 transcription factor silencing in mice.* To generate an experimental model where the endogenous Yy1 factor expression levels are silence, mice were transfected with a mixture of 4 specific interference RNA sequences (compounds 1 to 4 of Table 3). The infusion was made intravenously, through the tail vein of the animal, after 24 h of AMI. The siRNA sequences against Yy1 transcription factor (Custom siRNA, *in vivo* HPLC Accell) were acquired from Dharmacon (A-050273-13, -14, -15 and -16) and administered together in three independent doses of 6 mg/kg at days 1, 7 and 14 after AMI, that leads to a cumulative final concentration of 18 mg/kg. An outline of the experimental procedure is shown in Figure 2a. Briefly, just prior to use 75 µg of each one of the four siRNAs were mixed using Accell siRNA Delivery Media from GE Healthcare (B-005000). The target sequences both for the mouse-specific Yy1 transcription factor as well as a non-targeting Accell siRNA sequences (siCtrl), is shown in Table 3 below.

*LV structure and function.* Transthoracic echocardiography (2D and M-mode echocardiography) was performed on anaesthetized mice (1.8% isoflurane, inhalation) using a Vevo machine and a 13-MHz probe (MJFP), prior to surgery (baseline), 24 h post-AMI and 4 weeks post-AMI. From the four-chamber long axis views, the end-systolic (LVEsV) and end-diastolic left ventricular (LVEdV) volumes were determined by the Simpson method and the ejection fraction (EF) was determined automatically as EF (%) = LVEdV-LVEsV/LVEdV X 100. At the level of the chordae tendineae of the mitral valve, left ventricular end-diastolic (LVEdD) and end-systolic (LVEsD) dimension measurements were made by M-mode. Data are listed in Table 1. The investigators who analysed the data were blinded to the experimental groups.

**Table 1. Echocardiography measurements of sham and AMI control and siYy1 treated mice at 4 weeks post-AMI**

| Echocardiography parameters obtained from study | | | | | | |
|---|---|---|---|---|---|---|
| Treatment | Sham +PBS | Sham +siCtrl | Sham +siYy1 | AMI +PBS | AMI isiCtrl | AMI +siYy1 |
| N number | 12 | 10 | 10 | 10 | 10 | 9 |
| BW (g) | 24.03±0.13 | 24.1±0.32 | 24.62±0.41 | 25.1±0.12 | 25.20±0.15 | 25.20±0.14 |
| HR, bpm | 457.08±8.32 | 452±6.96 | 458.3±10.01 | 447.8±9.69 | 445.36±8.09 | 463±4.63*** |
| LVEDD (mm) | 4.82±0.08 | 4.61±0.15 | 4.89±0.12 | 4.74±0.54 | 4.81±0.04 | 4.43±0.22* |
| LVESD (mm) | 2.64±0.06 | 2.59±0.09 | 2.76±0.08 | 3.29±0.17 | 3.38±0.06### | 2.63±0.04*** |
| LVEDV (mL) | 71.01±0.35 | 71.15±0.41 | 71.41±3.31 | 110.22±3.54 | 105.45±5.22### | 93.53±3.46*** |
| LVESV (mL) | 35.12±0.74 | 36.14±0.52 | 31.87±0.33 | 68.58±0.69 | 68.45±0.88## | 51.45±3.33*** |
| EF (%) | 50.54±0.98 | 49.20±0.67 | 55.37±0.96 | 37.78±2.10 | 35.08±1.39### | 44.99±0.86*** |
| FS (%) | 45.22±1.25 | 43.82±0.65 | 43.56±1.54 | 30.59±2.32 | 29.73±1.56### | 40.63±1.56*** |

| | | | | | | |
|---|---|---|---|---|---|---|
| BW, body weight; HR, heart rate; LV, left ventricle; LVEDV, left ventricular end-diastolic volume; LVESV, left ventricular end-systolic volume; LVEDD, left ventricular dimensions at end diastole; LVESD, left ventricular dimensions at end systole; FS, fractional shortening; EF, ejection fraction. ###P<0.001 versus Sham+siCtrl, *p<0.05 and *** p<0.001 versus AMI+siCtrl. P values were calculated using unpaired, two-tailed Student's t-tests. Data represent means ± SEM. | | | | | | |

### RNA extraction and quantitative RT-PCR

Fresh infracted ventricle (~ 20 mg) were washed with cold DPBS. Then, samples were placed in a pre-chilled glass Petri dish and chopped in an ice bath using sharp scissors. RNA isolation and quantitative real-time PCR were performed according to the manufacturer's protocol with minor modifications(15). The primer sequences used for quantitative real-time PCR analysis are described in table 2.

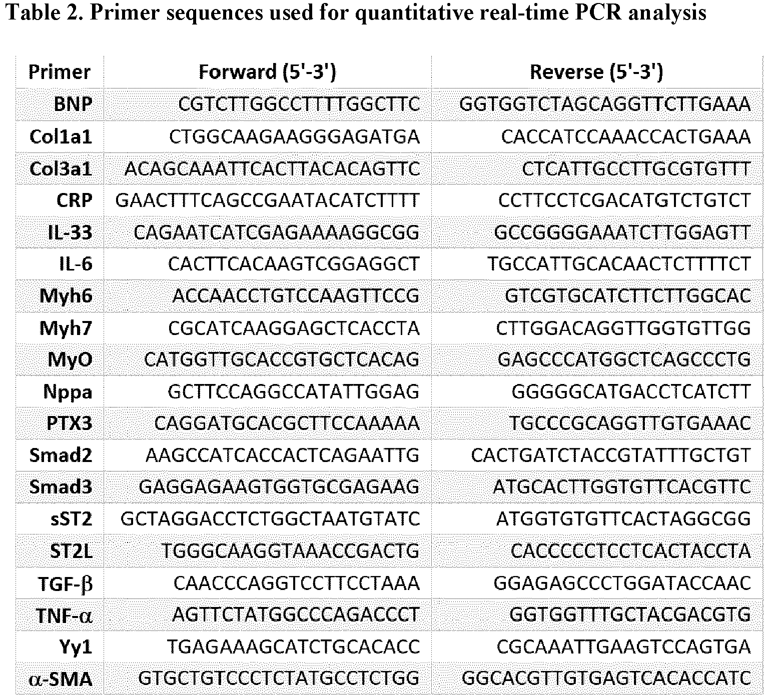

### Statistical analysis.

Data were expressed as mean ± standard error of the mean. Normality was tested using the Kolmogorov-Smirnov test. Differences between all groups were tested with a Kruskal-Wallis test. For multiple comparisons with the sham group, the Siegel-Castellan test was used. Non-parametric correlations were studied only in infarcted animals following Kendall's method. Statistical significance was assumed at p<0.05. Data were statistically analyzed using SPSS statistics 22 (IBM Corp., Armonk, NY, USA). Graphing was performed using SigmaPlot 11.0 software. P values of < 0.05 were considered statistically significant.

### Results

### AMI induces an up-regulation of Yin yang-1 (Yy1) transcription factor

First, we quantified Yy1 levels in total RNA extracted from left ventricle (LV) of infarct and non-infarct mice (Figure 1). As shown in Figure 1a, Yy1 mRNA levels in the LV infarct area increased significantly **after one week from AMI** (p<0.01); an increase that was higher when the time period was prolonged to 4 weeks (p<0.001) (Figure 1a). The increase on Yy1 mRNA did not occur in the remote LV area (Figure 1b).

### Genetic deficiency of Yy1 transcription factor prevents adverse cardiac remodeling following AMI

Next, starting from the observation that decreased Yy1 levels and activity by specific siRNAs, prevented hypertrophy of neonatal cardiomyocytes *in vitro*(15), we asked whether a global genetic deletion of Yy1 prevents the pathologic cardiac remodeling following AMI. As shown in figure 2a, and as described above, siYy1 therapy was initiated after AMI induction; immediately after ST segment elevation. When we evaluated Yy1 mRNA levels in the infarcted LV area (AMI+siCtrl group), as compared to mRNA levels obtained in sham group (Sham+siCtrl group), we observed a significant increase (p<0.001) that was prevented when animals were treated with siYy1 (AMI+siYy1 vs. AMI+siCtrl; p<0.001) (Figure 2b).

Importantly, infarcted mice treated with siYy1 were protected from AMI-induced cardiac hypertrophy and functional impairment. Treated animals showed better heart function (Figure 3) (p<0.001), lower macroscopic cardiac hypertrophy (i.e., heart weight) (p<0.001) (Figure 4a), lower levels of hypertrophic-associated marker genes Myh7 (in relation to Myh6) and Nppa (p<0.001, in both cases) (Figure 4, b-c), and lower cardiac myocytes hypertrophy (p<0.01) (Figure 4d).

Remarkably, also myocardial fibrosis was reduced in these siYy 1-deficient mice, as determined by a fibrosis-associated marker genes deregulation (Figure 5, a-f). Sirius Red and Masson staining confirmed these protective results (Figure 5g).

Similar effects were obtained when we evaluated the effect of siYy1 therapy on cardiac inflammation following AMI. Again, siYy1 therapy prevented adverse inflammation following AMI in term of an inflammation-associated marker genes deregulation (Figure 6).

Next, we carried out experiments related to myocardial death. As shown in figure 7, AMI induces cell death of infarcted LV myocardium, which is characterized by a significant increase in the levels of MyO (p<0.001), H-FABP (p<0.001) and BNP mRNA levels (p<0.001) (Figure 7). siYy1 therapy prevented this increase (p<0.001, in all cases).

***The protective cardiac effect of siYy1 therapy is related to the modulation of IL-33*/*ST2 axis.***

Next, to confirm whether the beneficial effect of siYy1 therapy during cardiac remodeling *in vivo* is related with IL33/ST2L axis, the mRNA levels of IL-33, ST2L and sST2 we evaluated in the infracted LV area from mice by quantitative RT-PCR (Figure 8). AMI was associated with an increase in IL-33 (p<0.001), ST2L (p<0.001) and sST2 (p<0.01) mRNA expression. Interestingly, the lack of endogenous Yy1 had no effect on IL-33 and ST2L mRNA levels and prevented the up-regulation of myocardial sST2 mRNA level. The silencing of Yy1 in the absence of damage or the use of PBS, had no effect on IL33, ST2L and sST2 expression compared to their respective controls.

### Example 2.

### MATERIAL AND METHODS

Animal Care and ethical aspects. C57B1/6J male mice (weighing 25-30 g) were purchased from the Jackson Laboratory. Mice were housed in specific pathogen free environment with a relative humidity of 50 ± 5% at 23 ± 2 °C with 12 h light and dark cycles. Mice had free access to food and water. All animal experiments were approved by the Ethics Review committee for animal use at the University of Murcia (Permit number: A13150105). After 7 days' adaptation, the animals were subjected to a surgical procedure to induce MI before systemic Yy1 genetic silencing.

Induction of experimental MI in mice. Before the surgical procedures, animals were anesthetized with intraperitoneal ketamine (75 mg/kg) and medetomidine (0.5 mg/kg), before being intubated and ventilated with a 18 gauge intravenous catheter and placed in supine position over a temperature control pad. The animals were monitored by electrocardiogram (ECG) electrodes connected to the limbs through small needles inserted subcutaneously. Left-sided thoracotomy was performed by a small incision between the third and fourth intercostal spaces. The incision was expanded by a blunt ended retractor in such a manner that the lungs were avoided in the area of retraction. The pericardial sac surrounding the heart was cut open, but the heart was not exteriorized. The ligation site of the left anterior descending coronary artery (LAD) was determined 4 mm away from the origin. Using a tapered atraumatic needle, a 8-0 silk ligature was passed underneath the LAD and tied with three knots. Visible blanching and cyanosis of the anterior wall of the left ventricle and swelling of the left atrium were taken as indicative of successful ligation. The procedure was considered successful if the ECG showed ST-segment elevation and the anterior wall of the left ventricle became blanched. Ribs and muscles were closed using 6-0 vicryl dissolvable sutures, leaving a small gap to aspirate any air left in the chest cavity. The air was aspirated by an in-house tube (2 mm diameter), again without touching the lungs. At the time of closure, neomycin powder and betadine were applied to the muscle and skin stitch sites, respectively. The surgical site was dressed daily to avoid any infection and to monitor for any dehiscence of the suture site. The entire procedure was performed within 20 min of the induction of anesthesia. Sham-operated rats underwent the same procedure without any ligation. After surgery, the animals received four doses of buprenorphine (0.05 mg/kg, subcutaneous) at 8 h intervals. The sham groups underwent the same surgical procedure except that the LAD coronary artery was not occluded. Electrocardiographic monitoring was used to confirm ST segment elevation after MI induction. After surgery-induced MI, infarcted mice show significant changes in electrocardiogram compared to those of sham group (Upper panels in Figure 9). To evaluate the evolution of cardiac damage, an echocardiographic study was performed on each mouse at sacrifice (4- or 8-weeks following MI) (Sacks D. et al., Int J Stroke. 2018 Aug;13(6):612-632).

Experimental design and study protocol in mice. At 30 min post-MI, survival animals were randomized into two global groups: MI and Sham. In each case, animals were regrouped based on siYy1 therapy initiation (6 mg/kg, i.v.), which started after 1 hour (S1h), 24 hours (S24h), 3 days (S3d), 7 days (S7d) or 14 days (S14d) post-MI. SiYy1 therapy was repeated every 7 days until completing a total of 3 cycles each (Figure 9). The animals belonging to the groups S1h, S24h, S3d and S7d were sacrificed at 4 weeks post-MI, while those belonging to S14d were sacrificed at 8 weeks after MI (Scheme in Figure 9).

Human iPs-derived cardiomyocytes (hiPsCMs) preparation and biomechanical strain. Human induced pluripotent stem cells (hiPSC) were purchased from Phenocell (PCi-CAU); ~0.5 × 105 viable cells were provided in cryovials. Initially, on a suitable matrix to allow attachment of cell aggregates (Matrigel^{®}), hiPSC were grown to 80% confluence using mTeSR^{™} plus medium and maintained in a humidified atmosphere of 5% CO2 and 95% air at 37 °C. At passage 20, reprogramming procedure was started using a standardize protocol in our laboratory. Briefly, culture medium was removed and changed to RPMI-1640 with B27 minus insulin supplement (basal differentiation supplement) with 4 µM CHIR99021 (day 0). On day 3, medium was changed to basal differentiation supplement and 3 µM IWR-1. On day 5, medium was refreshed with basal differentiation supplement. Medium was changed on day 8 to RPMI 1640 with B27 supplement (cited above as RMPIB27). On day 11, medium was changed to RPMI 1640 without glucose with B27 minus insulin. On day 14, medium was changed to RPMI/B27. Cultures for the differentiation were maintained in a 5% CO2/95% air environment at 37 °C. During differentiation, the medium was replaced every 3 days. Beating clusters were observed after 20 days. HiPsCMs were allowed to recover for 12 days in iCell Maintenance Medium (Cellular Dynamics International) before experimentation. A standardizing gene expression profiling (RT-qPCR) both hiPSC as well as hiPsCMs was carried in each human isolated clone, before starting assays. OCT4 and NANOG mRNA levels were used as hiPSC-specific markers and the mRNA levels to cTnT and NKX 2-5, as iPsCMs-specific markers.. Biomechanical strain was performed using an adaptation of the experimental design described by Asensio-Lopez MC. et al. (Sci Rep. 2021 Feb 16;11(1):3915).Here, hiPsCMs were co-stimulated with 0.2 µM PMA and 0.4 µM A23187 for 6 h, thus inducing sustained cell stretching.

Endogenous Yin-Yang 1 transcription factor silencing. To generate an experimental model where Yy1 expression levels are down-regulated, mice were treated with a mixture of four specific interference RNA sequences (compounds 1 to 4) and hiPsCMs were transfected with an equivalent mixture (compounds 5 to 8) -specifically designed to human Yy1 mRNA sequence-.

**Table 3. Specific short interfering RNAs sequences to silent Yy1 levels.**

| |
|---|
| **Compound 1 (siRNA 1):** |
| Sense sequence: CUGUUGUCCAGAAUACUUAUU (SEQ ID NO 1) |
| Antisense sequence: 5 -PUAAGUAUUCUGGACAACAGUU (SEQ ID NO 2) |
| |
| **Compound 2 (siRNA 2):** |
| Sense sequence: CAUGUAGAAUCAAAUAUUAUU (SEQ ID NO 3) |
| Antisense sequence: 5 -PUAAUAUUUGAUUCUACAUGUU (SEQ ID NO 4) |
| |
| **Compound 3 (siRNA 3):** |
| Sense sequence: GCUCCAAGAACAAUAGCUUUU (SEQ ID NO 5) |
| Antisense sequence: 5 -PAAGCUAUUGUUCUUGGAGCUU (SEQ ID NO 6) |
| |
| **Compound 4 (siRNA 4):** |
| Sense sequence: CAACUAACCUGAAAUCUCAUU (SEQ ID NO 7) |
| Antisense sequence: 5'-PUGAGAUUUCAGGUUAGUUGUU (SEQ ID NO 8) |
| |
| **Compound 5 (siRNA 5):** |
| Sense sequence: CAU GUA GUA UCA AAU AUU A (SEQ ID NO 49) |
| Antisense sequence: 5 - UAA UAU UUG AUA CUA CAU G (SEQ ID NO 50) |
| |
| **Compound 6 (siRNA 6):** |
| Sense sequence: GGG AUA UGC UUA GUA AUG C (SEQ ID NO 51) |
| Antisense sequence: 5 - UAA UAU UUG AUA CUA CAU G (SEQ ID NO 52) |
| |
| **Compound 7 (siRNA 7):** |
| Sense sequence: CUC AGU UGU AGA AUG UAU U (SEQ ID NO 53) |
| Antisense sequence: 5 - UAA UAU UUG AUA CUA CAU G (SEQ ID NO 54) |
| |
| **Compound 8 (siRNA 8):** |
| Sense sequence: CAA CUA ACC UGA AAU CUC A (SEQ ID NO 55) |
| Antisense sequence: 5 - UAA UAU UUG AUA CUA CAU G (SEQ ID NO 56) |
| |
| **non-targeting Accell siRNA sequences (siCtrl/Scr):** |
| Sense: UAGCGACUAAACACAUCAAUU (SEQ ID NO 9) |
| Antisense: 5'-PUUGAUGUGUUUAGUCGCUAUU (SEQ ID NO 10) |

In mice, the infusion was made intravenously through the tail vein of the animal after 1h, 24h, 3d, 7d or 14d after MI. The siRNA sequences against Yy1 transcription factor (Custom siRNA, in vivo HPLC Accell) were acquired from Dharmacon (A-050273-13, -14, -15 and -16) and administered together in three independent doses of 6 mg/kg per week after MI. An outline of the experimental procedure is shown in Figure 9. Briefly, just prior to use 75 □g of each one of the four siRNAs were mixed using Accell siRNA Delivery Media from GE Healthcare (B-005000). The target sequences both for the mouse specific Yy1 transcription factor as well as a non-targeting Accell siRNA sequences (siCtrl), is shown in Table 3 above (cited as compound 1 to 4 and non-targeting Accell siRNA sequences (siCtrl).

In hiPsCMs, Yy1-specific siRNA or control siRNA (Dharmacon, Lafayette, CO) were transfected into cells using Lipofectamine RNAiMAX. To prevent off-target effects caused by both the sense and antisense strands, a commercial mixture of four individual duplexes designed to target one gene was used (compound 5 to 8 and Scr; Table 3). Briefly, hiPsCMs (~20 × 103 cells) were plated into a six-well plate and grown at 37 °C in complete medium for 2 days. Next, cells were subjected to transfection using Lipofectamine RNAiMAX (Invitrogen, Carlsbad, CA, USA) according to the manufacturer's instructions. The transfection concentration was 25 nM. Cells were maintained for 24 h after adding the transfection mix. Then, transfected cells were washed twice with DPBS at 37 °C and then co-stimulated with PMA and A23187 for 6h, as indicated previously.

LV structure and cardiac function. Transthoracic echocardiography (2D and M-mode echocardiography) was performed on anaesthetized mice (1.8% isoflurane, inhalation) using a VEVO machine and a 13-MHz probe, after 4- or 8-weeks post-MI. From the four-chamber long axis views, the end-systolic (LVEsV) and end-diastolic left ventricular (LVEdV) volumes were determined by the Simpson method and the ejection fraction (EF) was determined automatically as EF (%) = LVEdV-LVEsV/LVEdV× 100. At the level of the chordae tendineae of the mitral valve, left ventricular end-diastolic (LVEdD) and end-systolic (LVEsD) dimension measurements were made by M-mode. The investigators who analyzed the data were blinded to the experimental groups.

Tissue samples and histology. Four weeks (S1h, S24h, S3d and S7d groups) or 8 weeks (S14d groups) after the LAD artery ligation, animals were sacrificed, and their hearts were arrested in diastole by intravenous injection of 0.2 ml 10% (w/v) potassium chloride (MERCK, USA). Then, the hearts were excised and rinsed with ice cold DPBS before the removal of the right ventricle and the atria. For the histopathological analyses, mid-papillary slices of the left ventricle of seven mice from each treatment group were fixed in 4% formaldehyde up to 24 h before paraffin embedding. Sirius red staining was performed to evaluate fibrosis. For its quantification, at least six random pictures from the border infarcted zone were taken from each slide at 20× magnification. Collagen deposition (red) was used to define fibrosis, which is expressed as a percentage of red pixels to red pixels quantitated using DIGITAL IMAGE HUB software version 4.0.6. For other molecular-cellular biological studies, infarcted area was collected and stored at- 80 °C for RNA extraction. The observers who performed the images analyses, and the molecular and cellular biological experiments were blinded to the experimental groups.

Immunohistochemistry. The next experimental procedure was performed as previously described, with some modifications (Asensio-Lopez MC. et al., Sci Rep. 2021 Feb 16;11(1):3915). Briefly, the sections (3 µm) from paraffin-embedded mid-papillary slices of LV were placed on poly-l-lysine-coated glass slides. Then, the sections were de-paraffinized and pre-treated in DAKO PT Link for 20 min at 97 °C. For CD45 or caspase 3 staining, rehydrated sections were incubated overnight with a rabbit polyclonal CD45 antibody (working dilution 1:500, ABCAM [ab10558]) or with a rabbit polyclonal caspase 3 antibody (working dilution 1:300, CELL SIGNALLING [9661]). Next, the sections were incubated with anti-rabbit biotinylated-labelled polymer (DAKO ENVISION) according to the manufacturer's instructions and revealed with 2-2'diaminobencidine (DAB). A cytoplasmic dark-brown precipitate indicated a positive immunostaining. Images were captured using a Zeiss Axio Scope A10 (CARL ZEISS, Madrid, Spain) microscope. Six random pictures were taken of each slide at 20× magnification (n = 7 slices/each treated group).

RNA extraction and quantitative RT-PCR. Total RNA was isolated from the infarcted myocardial tissue samples as well as hiPsCMs under stretching. RNA was purified with the RNeasy Mini Kit (QIAGEN), and cDNA was prepared with the iScript cDNA Synthesis Kit (BIORAD LAB. INC., Madrid) according to the manufacturer's recommendation. Quantitative real time polymerase chain reaction (RT-qPCR) was performed with the TB Green Premix Ex Taq II (Tli RNase H Plus) Master Mix (TAKARA BIO INC., Europe). Glyceraldehyde 3-phosphate dehydrogenase (GAPDH) was used as housekeeping gene. Sequences of the used primers (MERCK, USA) are listed in additional Table 4.

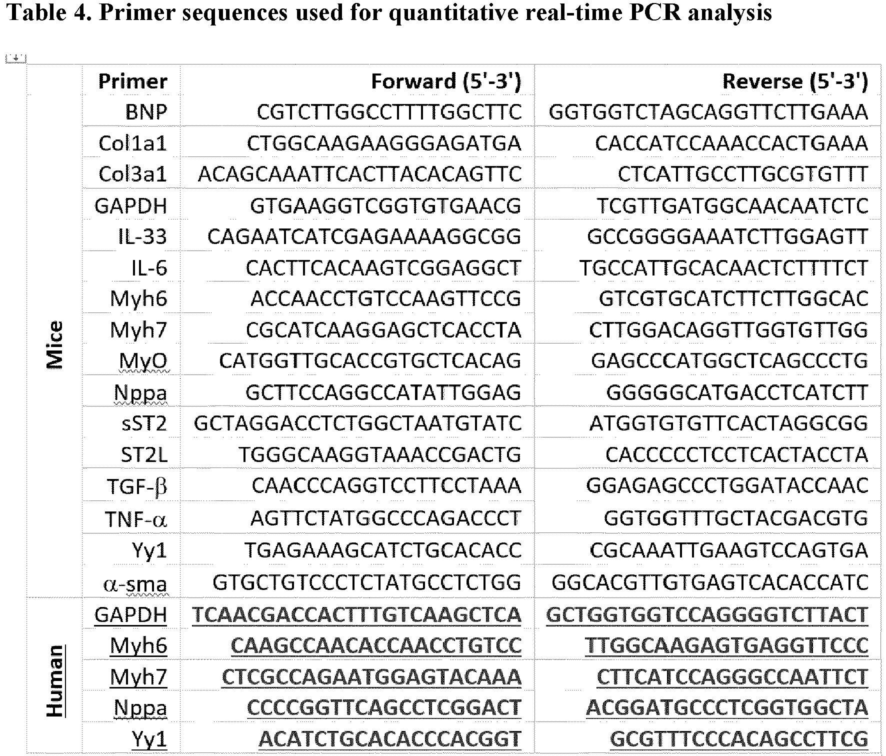

Statistical analysis. Linear regression models with interactions effects between infarction (MI vs. Sham), treatment (siYy1 vs. control) and time of initiation of treatment were performed to estimate means and standard error (SE). Due to imbalance of animals in each group marginal means were used (EMMs, estimated marginal means). The effect of infarction (changes between MI and Sham, MI - Sham) were estimated and reported in tables and figures. Differences in these changes (diff-in-diff) were also estimated for comparison and p-values calculations between treatments according to each starting time of therapy. A p value <0.05 were considered statistically significant. All analyses were performed using statistical software R (v.4.0) and package emmeans (v. 1.5.4).

### Results

The results for this example, are illustrated in figures 9 to 16. In this sense, it is herein noted that figure 11 shows that siYy1 therapy initiated within the first 3 days protected from MI-induced cardiac hypertrophy, in terms of macroscopic cardiac hypertrophy (a), HW/BW ratio (b). siYy1 therapy initiated within the first 7 days protected from MI-induced cardiac hypertrophy, in terms of levels of hypertrophic-associated marker genes Myh7 (related to Myh6) and Nppa (c). siYy1 therapy initiated at 14 days had not effect. Figure 12 shows that siYy1 therapy initiated within the first 3 days protected against LV systolic disfunction in terms of a significantly lower fall of LV ejection fraction (a) and fractional shortening (b). SiYy1 therapy initiated within the first 7 days protected against LV enlargement in terms of a lower increase in LV end-diastolic and end-systolic diameters (c, d), and LV end-diastolic and end-systolic volumes (e, f). siYy1 therapy initiated at 14 days had not effect. Moreover, and as shown in figure 13, siYy1 therapy initiated within the first 7 days protected against fibrosis in the infarcted myocardium, in terms of a significantly lower Sirius red staining (a) (representative images in panel b) as well as significantly lower levels of different fibrosis-associated marker genes deregulation (TGF-β, α-sma, Col1a1 and Col3a1 (c-f). Therapy initiated at 14 days had not effect. Figure 14 shows that siYy1 therapy initiated within the first 7 days protected against inflammation in the infarcted myocardium, in term of lower levels of CD45 positive staining (a) (representative images in panel b) and inflammation-associated specific markers (c-d). Therapy initiated at 14 days had not effect. Figure 15 shows that siYy1 therapy initiated within the first 7 days protected against cellular death in terms of a significantly lower increase of caspase 3 protein levels (a), as well as BNP and MyO mRNA levels (c-d). As shown in figure 16, siYy1 therapy prevented stretching-induced hypertrophy of human iPs-derived cardiomyocytes. Compared to control groups (Scr), the Yy1 mRNA levels in human iPs-derived cardiomyocytes (hiPsCMs) were elevated after stretching (PMA+Scr) (p<0.001) and were significantly reduced by siYy1 therapy (siYy1+PMA) (p<0.001) (a). siYy1 therapy protected from stretching-induced cardiac hypertrophy, in terms of levels of hypertrophic-associated marker genes Myh7 (related to Myh6) and Nppa (b, c).

## Claims

1. A composition comprising a compound capable of reducing the expression of the Yin Yang-1 (Yy1) gene in cardiac cells of a human or animal subject with respect to the expression observed in the absence of the compound in said cells, wherein the compound is a RNA interference (RNAi) of the Yy1 gene, and wherein said composition is for use in a method of treatment of left ventricular (LV) dysfunction following myocardial infarction (AMI) in the subject, and wherein said composition is administered between 12 hours and 7 days after the onset of myocardial infarction (AMI) in the subject.

2. The composition for use according to claim 1, wherein said composition is administered between 1 and 7 days after the onset of myocardial infarction (AMI) in the subject.

3. The composition for use according to claim 1, wherein said composition is administered between 12 hours and 3 days after the onset of myocardial infarction (AMI) in the subj ect.

4. The composition for use according to claim 1, wherein said composition is administered between 1 and 3 days after the onset of myocardial infarction (AMI) in the subject.

5. The composition for use according to claim 1, wherein said composition is administered between 3 and 7 days after the onset of myocardial infarction (AMI) in the subject.

6. The composition for use according to any of claims 3 or 4, wherein said composition is for use in a method of treatment of left ventricular (LV) dysfunction following myocardial infarction (AMI) in the subject by preventing, treating, mitigating, or reducing the loss of LV ejection fraction and/or fractional shortening.

7. The composition for use according to any of claims 3 or 4, wherein said composition is for use in a method of treatment of left ventricular (LV) dysfunction following myocardial infarction (AMI) in the subject by preventing, treating, mitigating, or reducing MI-induced cardiac hypertrophy.

8. The composition for use according to claim 5, wherein said composition is for use in a method of treatment of left ventricular (LV) dysfunction following myocardial infarction (AMI) in the subject by preventing, treating, mitigating, or reducing the LV enlargement.

9. The composition for use according to any of claims 1 to 8, wherein the interference RNA (RNAi) of the Yy1 gene is a siRNA selected from the list consisting of any of the following compounds: compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, compound 4 having sense SEQ ID NO 7 and antisense SEQ ID NO 8, compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56.

10. The composition for use according to any of claims 1 to 8, wherein the interference RNA (RNAi) of the Yy1 gene is a siRNA selected from the list consisting of any of the following compounds: compound 5 having sense SEQ ID NO 49 and antisense SEQ ID NO 50, compound 6 having sense SEQ ID NO 51 and antisense SEQ ID NO 52, compound 7 having sense SEQ ID NO 53 and antisense SEQ ID NO 54, and compound 8 having sense SEQ ID NO 55 and antisense SEQ ID NO 56.

11. The composition for use according to any of claims 1 to 10, wherein the compound is administered intravenously.

12. The composition for use according to any of claims 1 to 8, wherein the composition is a pharmaceutical composition comprising a therapeutically effective amount of an siRNA as defined in of claims 9 or 10 or vectors which express these oligonucleotides and a pharmaceutically acceptable carrier.

## Patentansprüche

1. Zusammensetzung umfassend eine Verbindung, welche in der Lage ist, die Expression des Yin Yang-1 (Yy1)-Gens in kardialen Zellen eines menschlichen oder tierischen Individuums in Bezug auf die Expression, welche in Abwesenheit von der Verbindung in den genannten Zellen beobachtet wird, zu verringern, wobei die Verbindung eine Interferenz-RNA (RNAi) des Yy1-Gens ist, und wobei die genannte Zusammensetzung für deren Verwendung in einem Behandlungsverfahren einer linksventrikulären (LV) Dysfunktion nach einem Myokardinfarkt (AMI) im Individuum ist, und wobei die genannte Zusammensetzung zwischen 12 Stunden und 7 Tagen nach dem Auftreten des Myokardinfarkts (AMI) im Individuum verabreicht wird.

2. Zusammensetzung für deren Verwendung nach Anspruch 1, wobei die genannte Zusammensetzung zwischen 1 und 7 Tagen nach dem Auftreten des Myokardinfarkts (AMI) im Individuum verabreicht wird.

3. Zusammensetzung für deren Verwendung nach Anspruch 1, wobei die genannte Zusammensetzung zwischen 12 Stunden und 3 Tagen nach dem Auftreten des Myokardinfarkts (AMI) im Individuum verabreicht wird.

4. Zusammensetzung für deren Verwendung nach Anspruch 1, wobei die genannte Zusammensetzung zwischen 1 und 3 Tagen nach dem Auftreten des Myokardinfarkts (AMI) im Individuum verabreicht wird.

5. Zusammensetzung für deren Verwendung nach Anspruch 1, wobei die genannte Zusammensetzung zwischen 3 und 7 Tagen nach dem Auftreten des Myokardinfarkts (AMI) im Individuum verabreicht wird.

6. Zusammensetzung für deren Verwendung nach einem der Ansprüche 3 oder 4, wobei die genannte Zusammensetzung für deren Verwendung in einem Behandlungsverfahren einer linksventrikulären (LV) Dysfunktion nach einem Myokardinfarkt (AMI) im Individuum ist, indem der Verlust der LV-Auswurffraktion und/oder fraktionellen Verkürzung vorgebeugt, behandelt, gelindert oder verringert wird.

7. Zusammensetzung für deren Verwendung nach einem der Ansprüche 3 oder 4, wobei die genannte Zusammensetzung für deren Verwendung in einem Behandlungsverfahren einer linksventrikulären (LV) Dysfunktion nach einem Myokardinfarkt (AMI) im Individuum ist, indem eine MI-induzierte Herzhypertrophie vorgebeugt, behandelt, gelindert oder verringert wird.

8. Zusammensetzung für deren Verwendung nach Anspruch 5, wobei die genannte Zusammensetzung für deren Verwendung in einem Behandlungsverfahren einer linksventrikulären (LV) Dysfunktion nach einem Myokardinfarkt (AMI) im Individuum ist, indem die LV-Vergrößerung vorgebeugt, behandelt, gelindert oder verringert wird.

9. Zusammensetzung für deren Verwendung nach einem der Ansprüche 1 bis 8, wobei die Interferenz-RNA (RNAi) des Yy1-Gens eine siRNA ausgewählt aus der Liste bestehend aus einer der folgenden Verbindungen ist: Verbindung 1 aufweisend Sense-SEQ ID NO 1 und Antisense-SEQ ID NO 2, Verbindung 2 aufweisend Sense-SEQ ID NO 3 und Antisense-SEQ ID NO 4, Verbindung 3 aufweisend Sense-SEQ ID NO 5 und Antisense-SEQ ID NO 6, Verbindung 4 aufweisend Sense-SEQ ID NO 7 und Antisense-SEQ ID NO 8, Verbindung 5 aufweisend Sense-SEQ ID NO 49 und Antisense-SEQ ID NO 50, Verbindung 6 aufweisend Sense-SEQ ID NO 51 und Antisense-SEQ ID NO 52, Verbindung 7 aufweisend Sense-SEQ ID NO 53 und Antisense-SEQ ID NO 54, und Verbindung 8 aufweisend Sense-SEQ ID NO 55 und Antisense-SEQ ID NO 56.

10. Zusammensetzung für deren Verwendung nach einem der Ansprüche 1 bis 8, wobei die Interferenz-RNA (RNAi) des Yy1-Gens eine siRNA ausgewählt aus der Liste bestehend aus einer der folgenden Verbindungen ist: Verbindung 5 aufweisend Sense-SEQ ID NO 49 und Antisense-SEQ ID NO 50, Verbindung 6 aufweisend Sense-SEQ ID NO 51 und Antisense-SEQ ID NO 52, Verbindung 7 aufweisend Sense-SEQ ID NO 53 und Antisense-SEQ ID NO 54, und Verbindung 8 aufweisend Sense-SEQ ID NO 55 und Antisense-SEQ ID NO 56.

11. Zusammensetzung für deren Verwendung nach einem der Ansprüche 1 bis 10, wobei die Verbindung intravenös verabreicht wird.

12. Zusammensetzung für deren Verwendung nach einem der Ansprüche 1 bis 8, wobei die Zusammensetzung eine pharmazeutische Zusammensetzung umfassend eine therapeutisch wirksame Menge einer siRNA nach einem der Ansprüche 9 oder 10 oder Vektoren, welche diese Oligonukleotide exprimieren, und einen pharmazeutisch akzeptablen Träger ist.

## Revendications

1. Composition comprenant un composé capable de réduire l'expression du gène Yin Yang-1 (Yy1) dans les cellules cardiaques d'un sujet humain ou animal par rapport à l'expression observée en l'absence du composé dans lesdites cellules, dans laquelle le composé est un ARN interférent (ARNi) du gène Yy1, et dans laquelle ladite composition est pour son utilisation dans une méthode de traitement du dysfonctionnement du ventricule gauche (VG) à la suite d'un infarctus du myocarde (IAM) chez le sujet, et dans laquelle ladite composition est administrée entre 12 heures et 7 jours après le début de l'infarctus du myocarde (IAM) chez le sujet.

2. . Composition pour son utilisation selon la revendication 1, dans laquelle ladite composition est administrée entre 1 et 7 jours après le début de l'infarctus du myocarde (IAM) chez le sujet.

3. . Composition pour son utilisation selon la revendication 1, dans laquelle ladite composition est administrée entre 12 heures et 3 jours après le début de l'infarctus du myocarde (IAM) chez le sujet.

4. . Composition pour son utilisation selon la revendication 1, dans laquelle ladite composition est administrée entre 1 et 3 jours après le début de l'infarctus du myocarde (IAM) chez le sujet.

5. . Composition pour son utilisation selon la revendication 1, dans laquelle ladite composition est administrée entre 3 et 7 jours après le début de l'infarctus du myocarde (IAM) chez le sujet.

6. . Composition pour son utilisation selon l'une quelconque des revendications 3 ou 4, dans laquelle ladite composition est pour son utilisation dans une méthode de traitement du dysfonctionnement du ventricule gauche (VG) à la suite d'un infarctus du myocarde (IAM) chez le sujet en prévenant, traitant, atténuant ou réduisant la perte de la fraction d'éjection du VG et/ou du raccourcissement fractionnel.

7. . Composition pour son utilisation selon l'une quelconque des revendications 3 ou 4, dans laquelle ladite composition est pour son utilisation dans une méthode de traitement du dysfonctionnement du ventricule gauche (VG) à la suite d'un infarctus du myocarde (IAM) chez le sujet en prévenant, traitant, atténuant ou réduisant l'hypertrophie cardiaque induite par l'IM.

8. . Composition pour son utilisation selon la revendication 5, dans laquelle ladite composition est pour son utilisation dans une méthode de traitement du dysfonctionnement du ventricule gauche (VG) à la suite d'un infarctus du myocarde (IAM) chez le sujet en prévenant, traitant, atténuant ou réduisant l'agrandissement du VG.

9. . Composition pour son utilisation selon l'une quelconque des revendications 1 à 8, dans laquelle l'ARN interférent (ARNi) du gène Yy1 est un pARNi sélectionné de la liste consistant en l'un quelconque des composés suivants : le composé 1 ayant SEQ ID NO 1 sens et SEQ ID NO 2 antisens, le composé 2 ayant SEQ ID NO 3 sens et SEQ ID NO 4 antisens, le composé 3 ayant SEQ ID NO 5 sens et SEQ ID NO 6 antisens, le composé 4 ayant SEQ ID NO 7 sens et SEQ ID NO 8 antisens, le composé 5 ayant SEQ ID NO 49 sens et SEQ ID NO 50 antisens, le composé 6 ayant SEQ ID NO 51 sens et SEQ ID NO 52 antisens, le composé 7 ayant SEQ ID NO 53 sens et SEQ ID NO 54 antisens, et le composé 8 ayant SEQ ID NO 55 sens et SEQ ID NO 56 antisens.

10. Composition pour son utilisation selon l'une quelconque des revendications 1 à 8, dans laquelle l'ARN interférent (ARNi) du gène Yy1 est un pARNi sélectionné de la liste consistant en l'un quelconque des composés suivants : le composé 5 ayant SEQ ID NO 49 sens et SEQ ID NO 50 antisens, le composé 6 ayant SEQ ID NO 51 sens et SEQ ID NO 52 antisens, le composé 7 ayant SEQ ID NO 53 sens et SEQ ID NO 54 antisens, et le composé 8 ayant SEQ ID NO 55 sens et SEQ ID NO 56 antisens.

11. . Composition pour son utilisation selon l'une quelconque des revendications 1 à 10, dans laquelle le composé est administré par voie intraveineuse.

12. Composition pour son utilisation selon l'une quelconque des revendications 1 à 8, dans laquelle la composition est une composition pharmaceutique comprenant une quantité thérapeutiquement efficace d'un pARNi selon les revendications 9 ou 10 ou des vecteurs qui expriment ces oligonucléotides et un véhicule pharmaceutiquement acceptable
